(19) Europäisches Patentamt
European Patent Office
Office européen des brevets

(11) EP 3 030 224 B1

(12) **EUROPEAN PATENT SPECIFICATION**

(45) Date of publication and mention of the grant of the patent:
**27.02.2019 Bulletin 2019/09**

(21) Application number: **14749773.9**

(22) Date of filing: **04.08.2014**

(51) Int Cl.:
***A61K 45/06*** *(2006.01)* ***A61K 31/439*** *(2006.01)*
***A61K 31/4704*** *(2006.01)* ***A61K 9/00*** *(2006.01)*
***A61K 9/16*** *(2006.01)*

(86) International application number:
**PCT/EP2014/066750**

(87) International publication number:
**WO 2015/018800 (12.02.2015 Gazette 2015/06)**

(54) **INHALABLE PARTICLES COMPRISING TIOTROPIUM AND INDACATEROL**

INHALIERBARE TEILCHEN MIT TIOTROPIUM UND INDACATEROL

PARTICULES INHALABLES CONTENANT DU TIOTROPIUM ET L'INDACATÉROL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.08.2013 EP 13382318**

(43) Date of publication of application:
**15.06.2016 Bulletin 2016/24**

(73) Proprietor: **Chemo Research, S.L.**
**28050 Madrid (ES)**

(72) Inventors:
- **AMIGHI, Karim**
  **1150 Brussels (BE)**
- **SERENO GUERRA, Antonio**
  **1820 Melsbroeck (BE)**
- **RONCHI, Celestino**
  **20124 Milano (IT)**
- **WAUTHOZ, Nathalie**
  **7500 Tournai (BE)**
- **HERNÁNDEZ HERRERO, Gonzalo**
  **28050 Madrid (ES)**

(74) Representative: **ZBM Patents - Zea, Barlocci & Markvardsen**
**Plaza Catalunya, 1 2nd floor**
**08002 Barcelona (ES)**

(56) References cited:
**WO-A2-2012/007729** **WO-A2-2013/021199**
**US-A1- 2004 132 759**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to inhalable particles comprising an intimate mixture which consists of

a) an amorphous tiotropium compound, b) an amorphous indacaterol compound, and c) optionally a sugar derivative, wherein the weight of the sugar derivative is comprised from 0 to 85% relative to the weight of the inhalable particles. It also relates to a pharmaceutical composition comprising the inhalable particles of the invention, to a process for their preparation, and to their use in the treatment of asthma or chronic obstructive pulmonary disease (COPD).

BACKGROUND ART

Tiotropium bromide was first disclosed in US 5610163 and has the following chemical structure:

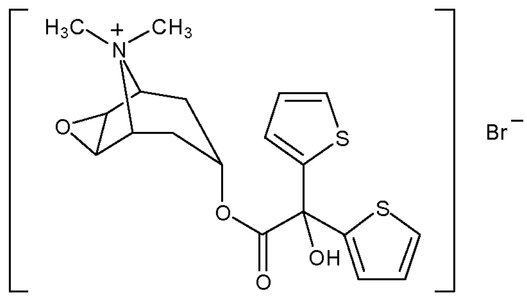

Different salts of this product (chloride, bromide, iodide, etc.) as well as different crystalline forms thereof are known. Tiotropium is a muscarinic receptor antagonist with highly effective anticholinergic effect. In the airways, it exhibits pharmacological effects through inhibition of M3-receptors at the smooth muscle leading to bronchodilation.

Indacaterol was first disclosed in US 6878721 and has the following chemical structure:

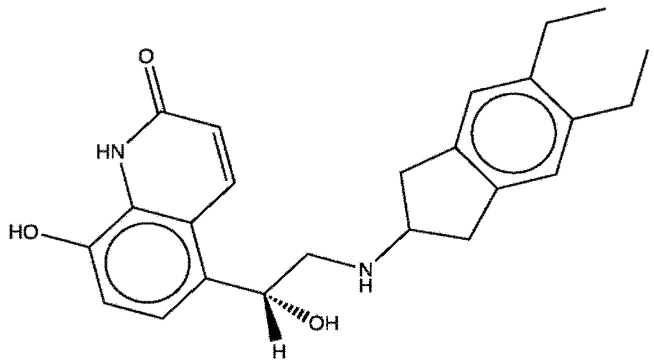

Indacaterol is a long-acting beta-2 adrenergic agonist (LABA), which causes bronchodilation by relaxing the smooth muscle in the airway and is intended for long-term maintenance treatment of inflammatory or obstructive airways diseases such as chronic obstructive pulmonary disease (COPD).

In patients whose COPD is not sufficiently controlled by monotherapy, therapy involving two long-acting bronchodilators with different mode of action is recommended. Combinations of long-acting muscarinic antagonists (LAMA) and long-acting beta-2 agonists (LABA) are probably those mostly studied in the literature and several once- or twice-daily fixed dose LAMA/LABA combinations are under development. Current evidence of beneficial therapeutic effect in COPD patients for LAMA/LABA combinations compared to monotherapy has been shown recently for different fixed-dose combinations such as tiotropium/formoterol, tiotropium/salmeterol, tiotropium/indacaterol, glycopyrrolate/formoterol, glycopyrronium/indacaterol at different dose ratios.

Several recent studies have shown that a beneficial therapeutic effect can be expected for the treatment of inflammatory or obstructive airways diseases from the concurrent use of tiotropium and indacaterol in comparison with a drug monotherapy.

For the treatment of respiratory diseases such as asthma or COPD, it is useful to administer the active substances by inhalation. In comparison to other inhalers, dry powder inhalers (DPI) offer flexibility in terms of nominal dose range, that is the amount of active substances that can be administered in a single inhalation, this making them especially

interesting as an administration tool. Thus, the use of inhalable powders containing active substances to be administered with a DPI is of particular importance.

Dry powder inhalation (DPI) products containing a drug combination are notoriously difficult to manufacture. Besides they have been reported to exhibit significant variability in the delivery of the active therapeutic agents present in the formulation, especially when the delivered drug doses are high and the ratio between the combined drugs is different. This can limit both the successful development of a DPI combination product and the synergistic action of the actives at the cellular level. This should be the case with combination products formulated as traditionally dry powders for inhalation, which consist in individually micronized drugs, blended with a coarse carrier particle or a mixture of fine and coarse carrier, typically lactose.

Such blends, which are generally of low-dose micronized active drug particles that attach themselves in a reversible fashion to the large carrier particles, are called ordered or interactive mixtures. This generally gives rise to a uniform and stable mixture. Nevertheless, lot of difficulties can be encountered in obtaining homogeneous mixtures, as the physico-chemical properties of the drugs and the excipients are very different (i.e. segregation of the active cohesive components as a function of mixing time or the mixer used). The problem is more critical when using a combination of two micronized drug rather than one single drug in the dry powder formulation. Moreover, batch to batch variabilities can also be observed from small variations of the physical properties of the micronized drugs and the coarse carrier.

This is also the case for the performances of the dry powder formulations such as the delivered doses from the drug powder inhaler or the aerodynamic performances. Indeed, the delivery efficiency of dry-powder products for inhalation is dependent upon the drug formulation, the inhaler device, and the inhalation technique. Concerning the composition of conventional dry powder formulations and the potential interactions of the components of the formulations, interparticulate interactions (both cohesion, i.e., drug-drug, and adhesion, i.e., drug-carrier) are dominated by physical forces of interaction such as van der Waals forces, electrostatic charges, capillary forces and mechanical interlocking. Other interactions can be determined by chemical forces, such as acid-base interaction forces and hydrogen bonding. The different interparticulate interactions between the drugs and the carrier are very complex and can be subjected to multiple variable factors. The order of magnitude of the physical forces could vary with particle size, shape, surface properties, the hardness of the adhering particle, surface roughness, and the relative humidity (RH). The magnitude of the adhesive force between the drugs and the carrier is proportional to the diameter of micronised particles and varies following the distance between micronised particles and the carrier (van der Waals forces and electrical charges) or following the surface tension of the liquid between particles (capillary forces). Moreover, in dry powder formulations for inhalation based on the combination of two drugs, the relative hydrophilicity/hydrophobicity of the drugs not only affected the interaction forces between the particles of the blend (and thus the aerodynamic performances of the formulation), but also the dissolution rate obtained for each drug. Indeed, the drug dissolution from the deposited particles into the lung would be much faster rate for a highly hydrophilic and hydrosoluble drug than for a hydrophobic and poorly water-soluble drug.

Document US 2004/0132759 describes inhalable powders of tiotropium and indacaterol for the treatment of inflammatory or obstructive diseases of the respiratory tract, particularly asthma and/or COPD. The disclosed inhalable powders are prepared by adding micronized active substances tiotropium and indacaterol to the excipient mixture. However, micronized drug compositions, generally show poor performance in terms of dispersibility and fine particle fraction which describes the inhalable part of a powder. Moreover, the micronization process is a poorly reproducible method which is highly affected by small variability on the starting material and which has the difficulty to obtain homogeneous mixtures of the active ingredients and the excipients since long and multiple blending steps are needed.

Document WO 2012/007729 discloses formulations comprising R (+) budesonide for the treatment and/or prevention of respiratory, inflammatory or obstructive airway diseases which may additionally comprise one or more bronchodilators such as tiotropium and indacaterol.

Document WO 2013/021199 discloses a pharmaceutical composition comprising a eutectic composition of two pharmacologically active ingredients which include, among others, beta2 agonists or anticholinergics, for delivery to the lung by inhalation.

Alternatively, other formulations containing both a muscarinic receptor antagonist and a long-acting beta-2 adrenergic agonist (LABA) have been described. For example, document WO 2012/106575 describes a dry powder formulation for inhalation containing spray-dried particles that comprise a core of a first active ingredient in crystalline form that is coated with a layer of a second active ingredient in amorphous form that is dispersed in a hydrophobic excipient. In particular, this document discloses dry powders comprising indacaterol maleate and glycopyrrolate as active ingredients, and distearoylphosphatidylcholine (DSPC), calcium chloride, and optionally trehalose as excipients. These formulations are manufactured by spray drying an emulsion-based feedstock prepared by mixing a vehicle emulsion, which is prepared in a two-step process by emulsifying perfluorooctyl bromide in an aqueous dispersion of DSPC containing dissolved $CaCl_2$, and a drug solution, which is prepared from micronized crystals of indacaterol maleate, glycopyrrolate and trehalose in an aqueous medium. However, the process for the preparation of such formulations is still complex and requires micronization processes and multiple mixing and homogenizing steps. Moreover, in the above

formulation, the kinetic of release of the two drugs occurs with different release rates: first the drug present in the coating part and second, the crystalline drug which constitutes the core part.

Thus, there is a need for further developing inhalable dry powder compositions comprising tiotropium and indacaterol which avoid the disadvantages of the prior art formulations and show improvements concerning the homogeneity of the powder mixtures, the powder flowability and/or the powder dispersion, the aerodynamic properties, and the release profiles of both active compounds.

SUMMARY OF THE INVENTION

Inventors have developed a dry powder formulation which comprises inhalable particles as defined by the claims consisting of amorphous tiotropium, amorphous indacaterol and optionally a sugar derivative, which is both physically stable against crystallization and chemically stable against degradation. The dry powder formulation of the invention has good aerodynamic properties when administered by using an appropriate dry powder inhaler. In particular, it shows a similar delivered dose form the inhaler, aerodynamic particle size distribution and Fine Particle Fraction (FPF) for both active ingredients. Further, the inhalable particles show a good release profile (i.e. closer dissolution profiles) for both active ingredients.

In addition, the inhalable powder compositions of the invention exhibit a high degree of homogeneity, and minor fluctuations in the dispersion properties, which is crucial in ensuring that the inhalable proportion of active substances are released reproducibly in constant amounts and with the lowest possible variability. The compositions have additionally good flow properties.

The above features are of particular importance in order to obtain a synergistic action of two drugs, such indacaterol and tiotropium, through fixed drug combinations. The administration of indacaterol and tiotropium from a homogeneous powder such as for example the one obtained by co-spray-drying a combination of both drugs is likely to enhance the chance of co-deposition in comparison to separate administrations of the individual active agents or co-administration of the drug from conventional dry powder formulations containing a mixture of the two micronized drugs with an appropriate excipient. Further, the co-spray dried powders show high aerosol performance and uniform deposition of the two drugs in the airways and the release profiles of the two drugs are closer than those obtained from physically mixed drugs, even though the solubility profiles of indacaterol (low water solubility) and tiotropium (high water solubility) are quite distinct.

Furthermore, the inhalable particles of the invention can be obtained by a simple and fast process suitable for industrialization unlike prior art formulations which are not completely in amorphous form and require complex and laborious process conditions for their preparation.

Therefore, a first aspect of the present invention refers to inhalable particles comprising an intimate mixture which consists of:

a) an amorphous tiotropium compound,
b) an amorphous indacaterol compound, and
c) a sugar derivative,

wherein the weight of the sugar derivative is comprised from 0 to 85% relative to the weight of the inhalable particles.

Another aspect of the invention relates to a pharmaceutical composition which comprises an effective amount of the inhalable particles as defined above together with one or more pharmaceutically acceptable excipients or carriers.

The inhalable particles of the invention can be conveniently prepared by an appropriate drying method from a solution of the active ingredients mixed with a solution or suspension of the sugar derivative, if present. Therefore, another aspect of the invention refers to a process for preparing inhalable particles comprising the following steps:

a) If a sugar derivative is present in the final particles, dissolving or dispersing the sugar derivative in a solvent system comprising one or more organic solvents and optionally water to form a solution or a suspension;
b) dissolving a tiotropium compound and an indacaterol compound in a solvent system comprising one or more organic solvents and optionally water;
c) If a sugar derivative is present in the final particles, mixing the solution or suspension of step a) and the solution of step b); and
d) spray-drying, freeze-drying or vacuum drying the solution or suspension of step b) or step c) to obtain the desired inhalable particles.

As previously described, the inhalable particles of the present invention as defined above, or the pharmaceutical compositions comprising them are useful for the treatment of certain respiratory diseases. Therefore, another aspect of the present invention relates to the inhalable particles as previously defined, for use as a medicament, in particular, for

use in the treatment of asthma or chronic obstructive pulmonary disease (COPD). Thus, this aspect relates to the use of the inhalable particles as previously defined, for the manufacture of a medicament for the treatment of asthma or COPD, and may also be formulated as a method for the treatment of asthma or COPD comprising administering an effective amount of the previously defined inhalable particles of the invention and one or more pharmaceutically acceptable excipients or carriers, in a subject in need thereof, preferably a human.

BRIEF DESCRIPTION OF THE DRAWINGS

FIG. 1 shows the XRPD pattern of the inhalable particles of Reference example 3 (spray-dried indacaterol-tiotropium-87%lactose, 2J2012C) at T=0 (A) and after storage for 3 months at 40 °C and 75% relative humidity (B).

FIG. 2 shows the XRPD pattern of the inhalable particles of Example 4 (spray-dried indacaterol-tiotropium-55%lactose, 9J2012B) at T=0 (A) and after storage for 6 months at 40 °C and 75% relative humidity (B).

FIG. 3 shows the XRPD pattern of the inhalable particles of Example 5 (spray-dried indacaterol-tiotropium, 9A2013A) at T=0 (A) and after storage for 3 months at 40 °C and 75% relative humidity (B).

FIG. 4 shows the XRPD pattern of the inhalable particles of Reference example 7 (spray-dried indacaterol particles, SI-3A) at T=0 (left) and after storage for 3 months at 40 °C and 75% relative humidity (right).

FIG. 5 shows the XRPD pattern of the inhalable particles of Reference example 8 (spray-dried tiotropium bromide methanol solvate, ST-3B) at T=0 (left) and after storage for 3 months at 60 °C and 30% relative humidity (right).

FIG. 6 shows the XRPD pattern of the inhalable particles of Example 11 (spray-dried particles of indacaterol-tiotropium-30% lactose, SITL30-3A) at T=0 (left) and after storage for 3 months at 40 °C and 75% relative humidity (right).

FIG. 7 shows the XRPD pattern of the inhalable particles of Example 10 (spray-dried particles of indacaterol-tiotropium-40% lactose, SITL40-3A) at T=0 (left) and after storage for 3 months at 40 °C and 75% relative humidity (right).

FIG. 8 shows SEM images for the inhalable particles of Reference example 3 (indacaterol-tiotropium-87%lactose) at T=0 (left) and after storage for 3 months at 40 °C and 75% relative humidity (right).

FIG. 9 shows SEM images for the inhalable particles of Example 4 (indacaterol-tiotropium-55%lactose) at T=0 (left) and after storage for 6 months at 40 °C and 75% relative humidity (right).

FIG. 10 shows SEM images for the inhalable particles of Example 5 (indacaterol-tiotropium) at T=0 (left) and after storage for 3 months at 40 °C and 75% relative humidity (right).

FIG. 11 shows SEM images for the inhalable particles of Example 11 (indacaterol-tiotropium-30% lactose, SITL30-3A) at T=0 (left) and after storage for 2 months at 40 °C and 75% relative humidity (right).

FIG. 12 shows SEM images for the inhalable particles of Example 10 (indacaterol-tiotropium-40% lactose, SITL40-3A) at T=0 (left) and after storage for 2 months at 40 °C and 75% relative humidity (right).

FIG. 13 shows the % of Cumulative release (R) of indacaterol and tiotropium in time (minutes) from 18 capsules of Example 21 (Dry Powder for Inhalation based on indacaterol and tiotropium, 24G2013A) impacted on the FSI filter (Dae < 5 μm; Fantasmino inhaler containing one HPMC capsules loaded with 25 ± 2 mg, 100 L/min during 2.4 seconds). The cumulative amount of indacaterol (C) and tiotropium (A) release of Example 21 was calculated and expressed as the percentage of the amount of indacaterol and tiotropium release after 180 minutes and compared with cumulative release of indacaterol from 10 capsules of Onbrez® (D) impacted on FSI filter in the same conditions or with cumulative release of tiotropium from 10 capsules of Spiriva® (B) impacted on FSI filter in the same conditions.

FIG. 14 shows the % of Cumulative release (R) of indacaterol and tiotropium in time (min) from 15 capsules of Example 22 (Dry Powder for Inhalation based on indacaterol and tiotropium, 24G2013B) impacted on the FSI filter

(Dae < 5 μm; Fantasmino inhaler containing one HPMC capsules loaded with 25 ± 2 mg, 100 L/min during 2.4 seconds). The cumulative amount of indacaterol (C) and tiotropium (A) release of Example 22 was calculated and expressed as the percentage of the amount of indacaterol and tiotropium release after 180 minutes and compared with cumulative release of indacaterol from 10 capsules of Onbrez® (D) impacted on FSI filter in the same conditions or with cumulative release of tiotropium from 10 capsules of Spiriva® (B) impacted on FSI filter in the same conditions.

DETAILED DESCRIPTION OF THE INVENTION

For the purposes of the invention the term "fine particle fraction" (FPF) describes the inhalable part of a powder and is the dose (expressed in weight %) of particles having an aerodynamic diameter inferior to 5 μm in relation to the nominal dose (Fine particle dose/loaded dose x 100) or to the recovered dose (Fine particle Dose/recovered dose × 100).The Fine Particle Dose (FPD) can be determined by the methods described in the USP and European Pharmacopoeia for the aerodynamic assessment of fine particle. In a powder for inhalation which is well dispersible the FPF is more than 20%, preferably more than 30%.

The term "mean aerodynamic particle diameter" also known as mass median aerodynamic diameter (MMAD) indicates the aerodynamic particle size at which 50% of the particles of the powder have a smaller aerodynamic diameter.

Unless stated otherwise herein, the percentages given within the scope of the present invention are always percent by weight.

According to the invention, a first aspect relates to inhalable particles comprising an intimate mixture which consists of: a) an amorphous tiotropium compound, b) an amorphous indacaterol compound, and c) a sugar derivative, wherein the weight of the sugar derivative is comprised from 0 to 85% relative to the weight of the inhalable particles.

In another embodiment, the inhalable particles consist of an intimate mixture which consists of an amorphous tiotropium compound, an amorphous indacaterol compound, and optionally a sugar derivative as defined above.

As used herein, the term "amorphous" refers to compositions which are substantially non-crystalline, more particularly compositions containing less than 10% crystalline fractions, even more particularly less than 5%, less than 2%, and less than 1% crystalline fractions. Typically the inhalable particles of the invention do not show a discernable X-ray diffraction pattern, more particularly when scanned from, for example, 2 to 70 degrees (2-theta).

The term "inhalable" means that the particles are suitable for pulmonary administration. Inhalable particles can be dispersed and inhaled by means of an inhaler, so that the particles are transported deep into the branches of the lungs and are able to develop a local or a systemic activity, optionally through the alveoli. Generally, the particle size of an inhalable particle is comprised up to 10 μm, more preferably from 0.5 to 10 μm, more preferably 0.5-6 μm.

As used herein the term tiotropium compound encompasses tiotropium as the free ammonium cation (also referred herein to as tiotropium base) as well as any pharmaceutically acceptable salt thereof.

The term indacaterol compound encompasses indacaterol as the free base (indacaterol base) as well as any pharmaceutically acceptable salt thereof. Any solid forms of tiotropium and indacaterol are also encompassed by the present invention.

The term "pharmaceutically acceptable salt" as used herein refers to any non-toxic salt which can be used for therapeutic purposes. The preparation of pharmaceutically acceptable salts of the compounds of the invention can be carried out by methods known in the art.

Non-limiting tiotropium salts which may be used within the scope of the present invention are those compounds which contain, for example, chloride, bromide, iodide, methanesulphonate, para-toluenesulphonate, benzenesulphonate or methyl sulphate. In a preferred embodiment, the tiotropium compound is tiotropium bromide.

Pharmaceutically acceptable salts of indacaterol include salts formed with pharmaceutically acceptable non-toxic acids, including inorganic and organic acids. Such acids include for instance hydrofluoric acid, hydrochloric acid, hydrobromic acid, hydroiodic acid, nitric acid, sulfuric acid, phosphoric acid, acetic acid, benzenesulfonic acid, benzoic acid, citric acid, ethansulfonic acid, fumaric acid, lactic acid, maleic acid, malic acid, mandelic acid, methanesulfonic acid, succinic acid, tartaric acid, p-toluensulfonic acid, xinafoic acid, and the like. In a preferred embodiment, the indacaterol compound is selected from indacaterol maleate, indacaterol acetate, and indacaterol xinafoate, more preferably indacaterol maleate.

In the inhalable particles of the invention any of the components (tiotropium, indacaterol and the sugar derivative, if present) act as stabilizing agent having a stabilizing effect on the other ones so that the particles are both chemically as well as physically stable. It means that the inhalable particles of the invention are more stable (i.e. show slower chemical degradation or crystallization rate) than if they would prepared without the component(s) that act as a stabilizing agent.

As mentioned above, the inhalable particles of the invention comprise an intimate mixture of amorphous tiotropium, amorphous indacaterol and the sugar derivative, if present.

The term "intimate mixture" as used herein refers to the physical relationship of the components tiotropium, indacaterol and the sugar derivative, if present, and means that the components of the particles are mixed at molecular

level and cannot be separated or unmixed at molecular level by general physical methods.

This particular structure of the inhalable particles in the form of intimate mixture is the result of the specific drying method used. Thus, the intimate mixture of the components is obtainable by spray-drying, freeze-drying or vacuum drying, preferably spray-drying, a solution or suspension of the components as described herein.

In an intimate mixture, as opposed to a simple blend or mechanical mixture obtained by mechanically mixing the components, the particles of each of the components are not distinguishable as such at molecular level e.g. under the microscope or in other ways (e.g. spectroscopic evaluation methods such as infra-red or Raman).

In a particular embodiment, the tiotropium compound, the indacaterol compound, and optionally the sugar derivative form a matrix in which the components are dispersed at molecular level. In this case, all components forming the matrix are in amorphous state. The particles having this structure are obtainable by drying a solution containing both the sugar derivative, if present, and the active ingredients.

In another particular embodiment, the particles of the invention comprises a sugar derivative and the active ingredients are dispersed at the surface of the sugar derivative (i.e. sugar derivative, which may be in a crystalline state, is coated by a thin layer of the active ingredients in amorphous form). The particles having this structure are obtainable by drying a mixture of a suspension of the stabilizing agent and a solution of the active ingredients.

Thus, in the particles without the sugar derivative, the active ingredients are uniformly distributed within a matrix composed of both of the active ingredients dispersed at molecular level, and in the particles comprising the sugar derivative, the active ingredients are either uniformly distributed within a matrix in which the components are dispersed at molecular level, so that the molecules of active ingredients enter and settle in intermolecular spaces of the sugar derivative forming a solid solution, or alternatively, the active ingredients and optionally part of the sugar derivative are dispersed at molecular level at the surface of the sugar derivative.

In all the cases above, there is a straight contact between the components of the inhalable particles forming an intimate mixture, as opposed to the case of a simple blend obtained by mechanically mixing the components, wherein they are dispersed as individual particles or as agglomerates of particles.

The particular structure of the inhalable particles of the invention can be confirmed and distinguished from simple blends by well-known techniques such as Scanning electron microscopy (SEM) and/or X-Ray powder diffraction (XRPD).

In a preferred embodiment, the inhalable particles of the invention consist of an intimate mixture of an amorphous tiotropium compound and an amorphous indacaterol compound, which is free of sugar derivative.

In another preferred embodiment, the inhalable particles of the invention consist of an intimate mixture of an amorphous tiotropium compound, an amorphous indacaterol compound, and a sugar derivative, wherein the weight of the sugar derivative is comprised up to 85%, more preferably up to 75%, more preferably up to 65%, relative to the weight of the inhalable particles.

In another preferred embodiment, the inhalable particles of the invention consist of an intimate mixture of an amorphous tiotropium compound, an amorphous indacaterol compound, and a sugar derivative, wherein the weight of the sugar derivative is comprised from 15 to 85%, more preferably from 20 to 60%, relative to the weight of the inhalable particles.

In a preferred embodiment, the inhalable particles consist of a sugar derivative, and the weight of sugar derivative, the weight of the tiotropium compound (expressed as tiotropium base), and the weight of the indacaterol compound (expressed as indacaterol base) relative to the total weight of the inhalable particles is comprised from 20 to 85%, from 0.2 to 10%, and from 10 to 80% respectively.

In another preferred embodiment, the inhalable particles consist of a sugar derivative, and the weight of sugar derivative is comprised from 15 to 85%, more preferably from 20 to 60%, relative to the weight of the inhalable particles; the weight of the tiotropium compound (expressed as tiotropium base) is comprised from 0.1 to 25%, more preferably from 0.2 to 25%, even more preferably from 0.2 to 15%, relative to the weight of the inhalable particles; and the weight of the indacaterol compound (expressed as indacaterol base) is comprised from 5 to 90%, more preferably from 10 to 85%, relative to the total weight of the inhalable particles.

In another preferred embodiment, the inhalable particles are free of sugar derivative, and the weight of the tiotropium compound (expressed as tiotropium base) and the weight of the indacaterol compound (expressed as indacaterol base) relative to the total weight of the inhalable particles is comprised from 0.5 to 50% and from 50 to 99.5% respectively.

In another preferred embodiment, the inhalable particles are free of sugar derivative, and the weight of the tiotropium compound (expressed as tiotropium base) and the weight of the indacaterol compound (expressed as indacaterol base) relative to the total weight of the inhalable particles is comprised from 0.5 to 59% and from 41 to 99.5% respectively.

In another embodiment of the invention, the sugar derivative in the intimate mixture is selected from the group consisting of a monosaccharide, such as glucose or arabinose, a disaccharide, such as lactose, saccharose, maltose or trehalose, an oligo- or polysaccharide, such as dextrane or maltohexose, and a polyalcohol, such as sorbitol, mannitol,

or glycerol. In another embodiment of the invention, the sugar derivative is selected from the group consisting of a monosaccharide, such as glucose or arabinose, a disaccharide, such as lactose, saccharose, maltose or trehalose, an oligo- or polysaccharide, such as dextrane or maltohexose, and a polyalcohol, such as sorbitol, mannitol, xylitol, lactitol or maltitol. In a preferred embodiment, the sugar derivative is lactose (anhydrous or monohydrate).

In another embodiment of the invention, the residual moisture (i.e. water content in weight) in the particles is equal to or lower than 5%, preferably equal to or lower than 3%, more preferably equal to or lower than 2%, relative to the weight of the inhalable particles.

The term "residual moisture" in the particles refers to the content of water remaining in the inhalable particles after the bulk of the solvents has been removed during the process of their preparation. Thus, the residual moisture is measured directly after the preparation of the inhalable particles (i.e. at t=0) and can be conveniently measured by a gravimetric method well-known in the art based on the product weight loss between two different temperatures (e.g. 25 °C -125 °C or 25 °C -140 °C) during a drying cycle.

As mentioned above, the inhalable particles comprising an intimate mixture which consists of amorphous tiotropium, amorphous indacaterol and optionally a sugar derivative are both physically as well as chemically stable. This means that the active compounds do not suffer any physical transformation (i.e. from an amorphous to a crystalline form), and/or significant chemical degradation (e.g. by hydrolysis, oxidation or any other degradation mechanism), in particular within a period of 1 month, more preferably 3 months, more preferably 6 months.

Micron-sized particles tend to show strong adhesion and cohesion which in turn lead to poor flow properties and to powder aggregation. One approach used to enhance the powder flowability relates to the use of one or more non-inhalable carriers, i.e. coarse excipients having a particle size (e.g. 50-150 μm) which makes them not inhalable.

In some embodiments, at an appropriate dilution of the active compounds and an appropriate particle size of the sugar derivative, the inhalable particles of the invention already show acceptable flowability without the need of adding additional excipients, in particular coarse excipients.

For example, when the inhalable particles are obtained by drying a mixture of a suspension of a sugar derivative having a mean particle size of 1-9 μm, and a solution of the active compounds at an appropriate dilution, such as e.g. 0.2 to 15.0% of indacaterol and 0.02 to 0.8% of tiotropium, or alternatively, 0.1 to 2.4% of indacaterol and 0.012 to 0.144% of tiotropium, there is no more coarse excipient to add, and the mixing step is not required, so that good powder homogeneity is achieved.

In drugs such as indacaterol and tiotropium, which show a particularly high efficacy, only small amounts of the active drugs are needed in each single dose to achieve the desired therapeutic effect. As a consequence, in some embodiments, the active substances have to be diluted with one or more pharmaceutically acceptable excipients which are pharmacologically inactive substances in order to obtain dispersible particles and flowable powders. The dilution must be such that the amount applied from the powder inhaler exactly contains the desired dose. The excipients are not only used as fillers, but also for their ability of giving good flowability properties to the powder composition, facilitating thus the mixing process.

Thus, the invention relates to a pharmaceutical composition comprising an effective amount of the inhalable particles as previously defined together with one or more pharmaceutically acceptable excipients or carriers. The pharmaceutical compositions of the invention may be conveniently prepared by mixing the inhalable particles, which may be obtained by the process described below, and the excipients or carriers.

The expression "effective amount" as used herein, refers to the amount of inhalable particles that, when administered, is sufficient to prevent development of, or alleviate to some extent, one or more of the symptoms of the disease which is addressed. The particular dose of inhalable particles administered according to this invention will of course be determined by the particular circumstances surrounding the case, including the particular condition being treated, and other similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals (i.e. Gras - Generally recognized as safe - excipients) without excessive toxicity, irritation, allergic response, immunogenicity or other problems or complications commensurate with a reasonable benefit/risk ratio.

In one embodiment, the pharmaceutical composition comprises the inhalable particles of the invention and one or more coarse excipients having a mean particle size of 15 to 250 μm.

The sugar derivative contained within the particles in some embodiments of the invention and the coarse excipients may be based on chemically identical or different substances. In a particular embodiment, the stabilizing agent and the coarse excipient comprise the same chemical compound. More preferably, only one coarse excipient is used and it has preferably a mean particle size ranging from 15 to 250 μm, preferably from 50 to 150 μm. More preferably, the coarse excipient is lactose anhydrous or lactose monohydrate. These compositions show good flow and dispersion properties.

In a preferred embodiment of the invention, the weight of the indacaterol compound (expressed as indacaterol base) relative to the total weight of the pharmaceutical composition is comprised from 0.01 to 25%, preferably from 0.05 to 10%, more preferably from 0.1 to 5%, more preferably from 0.1 to 2.5%, more preferably from 0.1 to 2.4%, more preferably from 0.25 to 1.5%.

In another preferred embodiment of the invention, the weight of the tiotropium compound (expressed as tiotropium base) relative to the total weight of the pharmaceutical composition is comprised from 0.001-5%, preferably from 0.01 to 1%, more preferably from 0.01 to 0.5%, more preferably from 0.01 to 0.15%, more preferably from 0.012 to 0.144%, more preferably from 0.02 to 0.1%.

The ratios between indacaterol and tiotropium can be modified in the combination to obtain optimal synergistic action of the two drugs through different fixed drug combinations. In a preferred embodiment, the fixed drug combination products of the invention preferably comprises dosages from 25 μg to 600 μg, more preferably from 75 μg to 300 μg indacaterol (expressed in indacaterol base) and from 3 μg to 36 μg, preferably from 6 μg to 18 μg tiotropium (expressed in tiotropium base), more preferably 150 μg indacaterol (expressed in indacaterol base) and 18 μg tiotropium (expressed in tiotropium base) in a final pharmaceutical composition having a weight of 25 mg.

Preferably, the compositions of the invention are in the form of a capsule containing a powder for inhalation. The capsule may be formed of various materials, such as gelatine, cellulose derivatives, starch, starch derivatives, chitosan and synthetic plastics. In a preferred embodiment, the capsule is formed of cellulose derivatives. Non-limiting examples of such derivatives are hydroxypropylmethylcellulose (HPMC), hydroxypropylcellulose (HPC), methylcellulose (MC), hydroxymethylcellulose (HMC) or hydroxyethylcellulose (HEC). In a more preferred embodiment of the invention, the inhalation capsule comprises HPMC. In the most preferred embodiment, the HPMC capsules show a low residual humidity (water content below 4%).

As mentioned above the inhalable particles of the invention may be prepared by a simple and fast preparation process which comprises firstly forming a solution comprising the active ingredients indacaterol and tiotropium and optionally the sugar derivative in solution or suspension, and subsequently spray-drying, freeze-drying or vacuum drying the resulting solution or suspension.

In the process of the invention the active ingredients are always dissolved in a suitable solvent, whereas the sugar derivative, if present, may be in the form of a solution or a suspension.

In a particular embodiment, the step of dissolving the active compounds in the solvent system is carried out at a temperature comprised from room temperature (20-25 °C) to 30 °C, 40 °C, 50 °C or 60 °C.

The process of the invention has the advantage that avoids micronization steps by using a more reproducible process. Micronization is poorly reproducible and highly dependent on the physical properties of the starting materials. In the process of the present invention, the starting compounds indacaterol and tiotropium may be in any physical form or particle size, since they are firstly dissolved. The tiotropium compound may be in the form of any pharmaceutically acceptable salt or any pharmaceutically acceptable solvate thereof; and the indacaterol compound may be in the form of indacaterol base or a pharmaceutically acceptable salt thereof, or a pharmaceutically acceptable solvate either of indacaterol base or of a pharmaceutically acceptable salt thereof.

For the purposes of the invention, the term "solvate" refers to a molecular complex comprising a compound (tiotropium or indacaterol) or a salt thereof, and a stoichiometric or non-stoichiometric amount of one or more molecules of a pharmaceutically acceptable solvent such as water, methanol, ethanol and the like, bound by non-covalent intermolecular forces. When the solvent forming part of the molecular complex is water, the solvate is a hydrate.

In a preferred embodiment, the tiotropium compound is tiotropium bromide, more preferably tiotropium bromide monohydrate. In another preferred embodiment, the tiotropium compound is tiotropium bromide methanol solvate. In another preferred embodiment, the indacaterol compound is selected from indacaterol maleate, indacaterol acetate, and indacaterol xinafoate, more preferably indacaterol maleate.

Moreover, an advantage of the present method is that the inhalable particles of the invention give neutral pH values after dissolution in water. This is very interesting in term of lung tolerance as the neutral pH values of the lungs should not be modified after inhalation.

In one embodiment of the invention, if a sugar derivative is present in the final particles, the process for their preparation comprises the following steps:

a) dissolving or dispersing the sugar derivative in a solvent system comprising one or more organic solvents and optionally water to form a solution or a suspension;

b) dissolving a tiotropium compound and an indacaterol compound in a solvent system comprising one or more organic solvents and optionally water;

c) mixing the solution or suspension of step a) and the solution of step b); and

d) spray-drying, freeze-drying or vacuum drying the solution or suspension of step c) to obtain the desired inhalable particles.

In the above embodiment, the inhalable particles of the invention may be prepared either starting from a solution or a suspension of the sugar derivative in a suitable solvent.

In the case of starting from a solution of the sugar derivative, the sugar derivative is dissolved in a solvent system comprising one or more organic solvents and water in an amount which is sufficient to dissolve the sugar derivative so that a final limpid solution is obtained, and this solution is then mixed with the solution obtained from indacaterol and tiotropium compounds (solution of step b). As it will be obvious to a person skilled in the art, these steps may be carried out in any order to obtain the final solution comprising indacaterol, tiotropium and the sugar derivative. In this case, after applying the suitable drying method, the resulting particles comprise a matrix of the components in which the sugar derivative and the active ingredients are uniformly dispersed in a molecular level. In one embodiment, the ratio organic solvent/s and water which results in a limpid solution of the sugar derivative is from 1:4 to 4:1, more particularly is 1:1.

Alternatively, in the case of starting from a suspension of the sugar derivative, steps a) and b) are preferably carried out separately in any order, and more preferably, the sugar derivative is firstly dispersed and homogenized (for instance by using high speed or high pressure homogenization) in a solvent system comprising one or more organic solvents in the absence of water in order to obtain a homogeneous suspension of the sugar derivative, before adding the solution of the active ingredients (solution of step b). Alternatively, the solvent system for dispersing and homogenizing the sugar derivative may comprise water provided that the amount of water is not sufficient to dissolve the sugar derivative, so that a final suspension is also obtained. In one embodiment, the ratio organic solvent/s and water which results in a suspension of the sugar derivative is from 5:1 to 15:1, more particularly is 9:1. In this case, after applying the suitable drying method, the resulting particles comprise amorphous indacaterol and tiotropium dispersed in a molecular state at the surface of particles of the sugar derivative.

In a preferred embodiment, the sugar derivative is selected from the group consisting of a monosaccharide, a disaccharide, an oligo- or polysaccharide, and a polyalcohol, more preferably is anhydrous lactose or lactose monohydrate. In another preferred embodiment, the sugar derivative used for forming the starting suspension is preferably in the form of micronized particles having a mean particle size of 1-9 $\mu$m.

In another embodiment of the invention, if the particles are free of a sugar derivative, the process for their preparation comprises the following steps:

a) dissolving a tiotropium compound and an indacaterol compound in a solvent system comprising one or more organic solvents and optionally water; and
b) spray-drying, freeze-drying or vacuum drying the solution of step a) to obtain the desired inhalable particles.

As mentioned above, the active ingredients are always dissolved in a suitable solvent system, whereas the sugar derivative, if present, is dissolved in a suitable solvent system if the solvent contains water in an amount which is sufficient to dissolve the sugar derivative, or is dispersed in a suitable solvent system if the solvent either does not contain water or it contains water in an amount which is not sufficient to dissolve the sugar derivative.

In one embodiment, the organic solvents used in the process of the invention are ($C_1$-$C_4$)-alcohols or ($C_1$-$C_4$)-alkyl-($C_1$-$C_4$)-esters. The term "($C_1$-$C_4$)-alcohol" refers to a linear or branched alkyl chains comprising from 1 to 4 carbon atoms and one or more hydroxyl groups. This term includes, without limitation, methanol, ethanol, propanol, isopropanol and butanol. The term "($C_1$-$C_4$)-alkyl-($C_1$-$C_4$)-ester" refers to an linear or branched alkyl groups, comprising from 1 to 4 carbon atoms, substituted with an alkanoyloxy group comprising from 1 to 4 carbon atoms. Examples of ($C_1$-$C_4$)-alkyl-($C_1$-$C_4$)-esters include, without limitation, ethyl acetate, propyl acetate and butyl acetate. In a preferred embodiment of the invention, the organic solvent used is preferably a water miscible solvent. More preferably, the solvent is a ($C_1$-$C_4$)-alcohol, more preferably ethanol or isopropanol, and more preferably ethanol.

In a particular embodiment, the solvent system used for forming the solution of the active ingredients comprises one or more organic solvents, preferably a ($C_1$-$C_4$)-alcohol, and water; more particularly, the volume ratio of the organic solvent and water used is from 9:1 to 1:9, more particularly the volume ratio of the organic solvent and water used is 1:1.

In one embodiment, the ratio of solvent (including organic solvent and water) and solid ingredients (indacaterol + tiotropium and optionally sugar derivative) is comprised from 10:1 to 200:1, more particularly from 20:1 to 50:1 v/w (mL/g).

As it will be obvious to a person skilled in the art, the solution of this first step may be formed by mixing the solvents and the different components in any order.

The drying method used in the second step of the process for the preparation of the inhalable particles of the invention is spray-drying, freeze-drying or vacuum drying. For the purposes of the invention these drying methods are considered equivalent in the sense that they are alternative methods for the preparation of the amorphous matrix powder. These methods allow contributing to obtain a precise control of the mean particle size and particle size distribution, and also the improvement of the macroscopic powder properties such as bulk density, flowability, and dispersibility. In a preferred embodiment, the drying method used is spray-drying. A typical Spray-Dryer allows the recovery of a range of particle sizes going from 0.1 to 30 $\mu$m. The lower limitation is given by the separation ability of the cyclone used: smaller particles cannot be separated anymore and are going into the filter.

The inhalable particles obtainable by the preparation process described above also form part of the invention. Thus, the invention relates to inhalable particles as defined above obtainable by the process comprising the following steps:

a) If a sugar derivative is present in the final particles, dissolving or dispersing the sugar derivative in a solvent system comprising one or more organic solvents and optionally water to form a solution or a suspension;
b) dissolving a tiotropium compound and an indacaterol compound in a solvent system comprising one or more organic solvents and optionally water;
c) If a sugar derivative is present in the final particles, mixing the solution or suspension of step a) and the solution of step b); and
d) spray-drying, freeze-drying or vacuum drying the solution or suspension of step b) or step c) to obtain the desired amorphous inhalable particles.

For the purposes of the invention the expressions "obtainable", "obtained" and equivalent expressions are used interchangeably, and in any case, the expression "obtainable" encompasses the expression "obtained".

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

EXAMPLES

a) Preparation of inhalable particles by spray-drying from solutions of ethanol and water

The inhalable particles were prepared, at laboratory scale, by spray-drying using a Büchi Mini Spray Dryer B-290 (Büchi laboratory-Techniques, Switzerland). Different solutions were prepared in ethanol/water (1:1 v/v) at a temperature of 40 °C.

In the particles containing lactose, lactose monohydrate (Lactochem microfine 90% of the distribution of particles size inferior to 10 μm) was firstly dissolved in water and then ethanol was added with gentle stirring to obtain a clear solution. Afterwards, tiotropium and/or indacaterol were dissolved in the solvent medium. In the particles not containing lactose, tiotropium and indacaterol were dissolved in the solvent medium (water and ethanol). For spray-dried particles made from a lactose suspension, micronized lactose monohydrate (Lactohale LH300, 90% of the distribution of particles size inferior to 10 μm) in ethanol/water (9:1 v/v) was passed through a high speed homogenizer during 10 min at 24 000 rpm and then heated at 40°C. Then, tiotropium and then, indacaterol in solution were added under magnetic stirring in this suspension.

The preparations were then spray-dried with constant stirring. The following conditions were used during spray-drying: spraying air flow, 600 L/h; drying air flow, 35 $m^3$/h; solution/suspension feed rate, 3.5-4.0 g/min; nozzle size, 0.7 mm. The inlet temperature was established at 130 °C or 140 °C and, in these conditions, the outlet temperature varied between 61 °C and 68 °C. The resultant powder was blown through the cyclone separator and collected in a container.

The following conditions were also used during spray-drying: spraying air flow, 800 L/h; drying air flow, 35 $m^3$/h; solution/suspension feed rate, 3.5-4.0 g/min; nozzle size, 0.7 mm. The inlet temperature was established between 130°C and 150 °C and, in these conditions, the outlet temperature varied between 60 °C and 72 °C.

Powders were stored in High Density Polyethylene (HDPE) well closed containers under accelerated storage conditions (temperature of 40°C and relative humidity (RH) of 75%) and/or under high temperature storage conditions (temperature of 60°C and relative humidity (RH) of 30%). Alternatively, powders were stored in open containers.

Following this process the following examples were obtained:

Reference example 1: Spray-dried indacaterol particles

| | |
|---|---|
| Indacaterol maleate | 600 mg |
| Ethanol | 60 mL |

(continued)

| | |
|---|---|
| Water | 60 mL |

Total solid residue: 0.600 g (0.5% w/v), indacaterol maleate representing 100% w/w of the spray-dried powder initial solid residue. The inlet temperature was set at 130 °C. The corresponding stability batches 2J2012B and 12J2012 were stored in closed containers with desiccant at 40 °C and 75% RH and 26C2013A in closed containers with desiccant at 60 °C and 30%RH.

Reference example 2: Spray-dried particles of indacaterol and lactose (96.2%)

| | |
|---|---|
| Indacaterol maleate | 0.38872 g |
| Lactose monohydrate | 9.920 g |
| Ethanol | 100 mL |
| Water | 100 mL |

Total solid residue: 10.309 g (5.2% w/v), spray-dried residue containing combined indacaterol maleate (3.8% w/w) and lactose monohydrate (96.2% w/w). The inlet temperature was set at 130°C. The corresponding stability batch 9J2012A was stored in closed containers with desiccant at 40 °C and 75%RH.

Reference example 3: Spray-dried particles of indacaterol, tiotropium and lactose (87%)

| | |
|---|---|
| Indacaterol maleate | 0.300 g |
| Tiotropium bromide monohydrate | 0.360 g |
| Lactose monohydrate | 4.440 g |
| Ethanol | 100 mL |
| Water | 100 mL |

Total solid residue: 5.100 g (2.55% w/v), spray-dried residue containing combined indacaterol maleate (5.9% w/w), tiotropium bromide monohydrate (7.1% w/w) and lactose monohydrate (87.0% w/w). The inlet temperature was set at 130 °C. The corresponding stability batch 2J2012C was stored in closed containers with desiccant at 40 °C and 75% RH.

Example 4: Spray-dried particles of indacaterol, tiotropium and lactose (55.6%)

| | |
|---|---|
| Indacaterol maleate | 1.943 g |
| Tiotropium bromide monohydrate | 0.220 g |
| Lactose monohydrate | 2.712 g |
| Ethanol | 50 mL |
| Water | 50 mL |

Total solid residue: 4.875 g (4.875% w/v), spray-dried residue containing combined indacaterol maleate (39.9% w/w), tiotropium bromide monohydrate (4.5% w/w) and lactose monohydrate (55.6% w/w). The inlet temperature was set at 130 °C. The corresponding stability batches 9J2012B and 26C2013B were stored in closed containers with

desiccant at 40 °C and 75% RH and at 60°C and 30%RH, respectively.

Example 5: Spray-dried indacaterol-tiotropium particles

| | |
|---|---|
| Indacaterol maleate | 1.943 g |
| Tiotropium bromide monohydrate | 0.220 g |
| Ethanol | 50 mL |
| Water | 50 mL |

Total solid residue: 2.163 g (2.163% w/v), spray-dried residue containing combined indacaterol maleate (89.8% w/w) and tiotropium bromide monohydrate (10.27% w/w). The inlet temperature was set at 130°C. The corresponding stability batches 9A2013A and 26C2013C were stored in closed containers with desiccant at 40 °C and 75%RH and at 60 °C and 30%RH, respectively.

Reference example 6: Spray-dried particles of indacaterol and lactose (96.2%)

| | |
|---|---|
| Indacaterol maleate | 0.38872 g |
| Lactose monohydrate | 9.920 g |
| Ethanol | 100 mL |
| Water | 125 mL |

Total dry residue: 10.30872 g (4.58% w/v), spray-dried residue containing combined indacaterol maleate (3.8% w/w) and lactose monohydrate (96.2% w/w). The inlet temperature was set at 140°C. The corresponding stability batches 9A2013B and 26C2013D were stored in closed containers with desiccant at 40 °C and 75%RH and at 60 °C and 30%RH, respectively.

Reference example 7: Spray-dried indacaterol particles

| | |
|---|---|
| Indacaterol maleate | 4.2759 g |
| Ethanol | 50 mL |
| Water | 50 mL |

Initial solid residue: 4.2759 g (4.2759% w/v), indacaterol maleate representing 100% w/w of the initial solid residue. The outlet temperature was set at 70 °C. The corresponding stability batches SI-3A and SI-3B were stored in closed containers with desiccant at 40 °C and 75%RH and at 60 °C and 30%RH, respectively.

Reference example 8: Spray-dried tiotropium particles

| | |
|---|---|
| Tiotropium bromide methanol solvate | 4.2759 g |
| Ethanol | 50 mL |
| Water | 50 mL |

Initial solid residue: 4.2759 g (4.2759% w/v), tiotropium bromide methanol solvate representing 100% w/w of the initial solid residue. The outlet temperature was set at 70 °C. The corresponding stability batches ST-3A and ST-3B were stored in closed containers with desiccant at 40 °C and 75%RH and at 60 °C and 30%RH, respectively.

Example 9: Spray-dried particles of indacaterol, tiotropium and lactose (50%)

| | |
|---|---|
| Indacaterol maleate | 1.921 g |
| Tiotropium bromide methanol solvate | 0.229 g |
| Lactose monohydrate | 2.15 g |
| Ethanol | 50 mL |
| Water | 50 mL |

Initial solid residue: 4.3 g (4.3% w/v), containing combined indacaterol maleate (44.7% w/w), tiotropium bromide methanol solvate (5.3% w/w) and lactose monohydrate (50% w/w). The outlet temperature was set at 70 °C. The corresponding stability batches SITL50-3A and SITL50-3B were stored in closed containers with desiccant at 40 °C and 75%RH and at 60 °C and 30%RH, respectively.

Example 10: Spray-dried particles of indacaterol, tiotropium and lactose (40%)

| | |
|---|---|
| Indacaterol maleate | 2.306 g |
| Tiotropium bromide methanol solvate | 0.274 g |
| Lactose monohydrate | 1.72 g |
| Ethanol | 50 mL |
| Water | 50 mL |

Initial solid residue: 4.3 g (4.3% w/v), containing combined indacaterol maleate (53.6% w/w), tiotropium bromide methanol solvate (6.4% w/w) and lactose monohydrate (40% w/w). The outlet temperature was set at 70 °C. The corresponding stability batches SITL40-3A and SITL40-3B were stored in closed containers with desiccant at 40 °C and 75%RH and at 60 °C and 30%RH, respectively.

Example 11: Spray-dried particles of indacaterol, tiotropium and lactose (30%)

| | |
|---|---|
| Indacaterol maleate | 2.690 g |
| Tiotropium bromide methanol solvate | 0.320 g |
| Lactose monohydrate | 1.29 g |
| Ethanol | 50 mL |
| Water | 50 mL |

Initial solid residue: 4.3 g (4.3% w/v), containing combined indacaterol maleate (62.6% w/w), tiotropium bromide methanol solvate (7.4% w/w) and lactose monohydrate (30% w/w). The outlet temperature was set at 70 °C. The corresponding stability batches SITL30-3A and SITL30-3B were stored in closed containers with desiccant at 40 °C and 75%RH and at 60 °C and 30%RH, respectively.

Example 12: Spray-dried particles of indacaterol, tiotropium and lactose (20%)

| | |
|---|---|
| Indacaterol maleate | 3.074 g |
| Tiotropium bromide methanol solvate | 0.366 g |
| Lactose monohydrate | 0.86 g |
| Ethanol | 50 mL |
| Water | 50 mL |

Initial solid residue: 4.3 g (4.3% w/v), containing combined indacaterol maleate (71.5% w/w), tiotropium bromide methanol solvate (8.5% w/w) and lactose monohydrate (20% w/w). The outlet temperature was set at 70 °C. The corresponding stability batches SITL20-3A and SITL20-3B were stored in closed containers with desiccant at 40 °C and 75%RH and at 60 °C and 30%RH, respectively.

Example 14: Spray-dried indacaterol and tiotropium particles

| | |
|---|---|
| Indacaterol maleate | 3.88718 g |
| Tiotropium bromide methanol solvate | 0.46267 g |
| Ethanol | 50 mL |
| Water | 50 mL |

Initial solid residue: 4.3 g (4.3% w/v), containing combined indacaterol maleate (89.36% w/w), tiotropium bromide methanol solvate (10.64% w/w). The outlet temperature was set at 70 °C. The corresponding stability batches SIT-3A and SIT-3B were stored in closed containers with desiccant at 40 °C and 75%RH and at 60 °C and 30%RH, respectively.

Reference example 15: Spray-dried indacaterol and tiotropium around micronized lactose particles

| | |
|---|---|
| Indacaterol maleate | 97.18 mg |
| Tiotropium bromide methanol solvate | 11.57 mg |
| Lactose monohydrate micronized | 12.4 g |
| Ethanol | 90 mL |
| Water | 10 mL |

Initial solid residue: 12.50875 g (12.50875% w/v), containing combined indacaterol maleate (0.78% w/w), tiotropium bromide methanol solvate (0.09% w/w) and micronized lactose monohydrate (99.13%). The outlet temperature was set at 70 °C. The corresponding stability batches L99SIT-3A and L99SIT-3B were stored in closed containers with desiccant at 40 °C and 75%RH and at 60 °C and 30%RH, respectively.

Reference example 16: Raw micronized indacaterol maleate

Stability batches AN018 and C1148 were stored in closed containers with desiccant at 40 °C and 75%RH and at 60 °C and 30%RH, respectively.

Reference example 17: Raw indacaterol maleate (non micronized)

Stability batches RI-A and RI-B were stored in closed containers with desiccant at 40 °C and 75%RH and at 60 °C and 30%RH, respectively.

Reference example 18: Raw tiotropium bromide methanol solvate

Stability batches RTMeOH-A and RTMe-OH-B were stored in closed containers with desiccant at 40 °C and 75%RH and at 60 °C and 30%RH, respectively.

Example 19: Spray-dried particles of indacaterol and tiotropium

| | |
|---|---|
| Indacaterol maleate | 1.944 g |
| Tiotropium bromide monohydrate | 0.225 g |
| Ethanol | 50 mL |
| Water | 50 mL |

Batch SIT-3 stored in closed containers with desiccant at 25 °C and 60%RH.

Example 20: Spray-dried particles of indacaterol, tiotropium and lactose (55.6%)

| | |
|---|---|
| Indacaterol maleate | 1.944 g |
| Tiotropium bromide monohydrate | 0.225 g |
| Lactose monohydrate | 2.712 g |
| Ethanol | 50 mL |
| Water | 50 mL |

Batch SITL55-3 stored in closed containers with desiccant at 25 °C and 60% RH.

b) Preparation of dry powder for inhalation based on inhalable particles of indacaterol and tiotropium

The inhalable particles of Example 19 and Example 20 were blended with a laboratory scale well-known three-dimensional motion mixer (Turbula, Bachofen AG, Switzerland) with different types of lactoses, used as carrier, in order to obtain a final content of 150 μg of indacaterol as it is the case for the reference product Onbrez® 150 μg and 18 μg of tiotropium as it is the case for the reference product Spiriva® 18 μg, both used a comparator. Different Carrier-spray-dried inhalable particle mixtures were prepared and compared to the different reference products. Both materials (inhalable particle formulations and lactose carrier) were sieved (0.315 mm) prior the mixing step. A glass vessel was filled to 50% of its inner volume and the blending (about 25 g) was done at a mixing speed of 46.2 rpm in the Turbula mixer for 5 minutes. Then, the pre-mixing was sieved through the same sieve (0.315 mm) and then blended again in the Turbula mixer for 10 minutes. Then, 25 ± 2 mg of Indacaterol-Tiotropium DPI formulation was pre-weighted in HPMC N°3 capsules and stored in a well closed container in a dessicator in a controlled temperature and relative humidity (RH) conditions (25 °C - 60%RH).

Example 21: Dry powder for inhalation based on spray-dried inhalable particles of indacaterol and tiotropium

| | |
|---|---|
| Inhalable particles SIT-3 | 0.2169 mg |

(continued)

| | |
|---|---|
| Lactose monohydrate carrier | 24.7831g |

Batch 24G2013A stored in closed containers in a desiccator at 25°C, 60%RH.

Example 22: Dry powder for inhalation based on spray-dried inhalable particles of indacaterol, tiotropium and lactose (55.6%)

| | |
|---|---|
| Inhalable particles SITL55-3 | 0.4881 mg |
| Lactose monohydrate carrier | 24.5119 g |

Batch 24G2013B stored in closed containers in a desiccator at 25°C, 60%RH.

Evaluation of the physical stability (crystallinity-amorphous)

Differential Scanning Calorimetry (DSC)

DSC analysis was performed in order to characterize thermal properties of the formulations and the ingredients used (crystallinity, anhydrous state, etc.). A modulated-DSC apparatus MDSC Q2000 (TA Instruments, USA) was used. About 2 mg was weighed in Tzero pan and lid to be heated at 5 °C/min from 25 °C to 250 °C.

The results of DSC curve of Reference example 16 (raw indacaterol maleate, AN018) showed an endothermic peak at 190 °C (data not shown) corresponding to the melting point with decomposition of indacaterol maleate. The DSC curve of Reference example 1 (spray-dried indacaterol particles, 2J2012B) at T=0 showed an endothermic peak at 170°C (data not shown) corresponding at a premature melting-decomposition point. The glass transition and the exothermic peak corresponding to recrystallization of amorphous indacaterol were not detected.

Thermogravimetric analysis (TGA)

TGA analysis was performed in order to characterize thermal properties and residual humidity of the formulations and the ingredients used (water content, anhydrous state). A TGA Q500 Hig. Res. equipment (TA Instruments, USA) was used for this purpose. Runs in triplicate (about 5-10 mg of sample) were set with platinum pan from 25 °C to 150 or 300°C at a heating rate of 10 °C/min. The moisture level was determined by the weight loss obtained between 25 °C and 125 °C or 25 °C and 140 °C.

The results presented in Table 1 show the moisture content determined by % of weight loss between 25 °C-125 °C for particles of Reference example 1 (12J2012=2J2012B), Reference example 2 (9J2012A), Reference example 3 (2J2012C), and Example 4 (9J2012B) at t=0, and after 15 days, 1 month, 3 months, and 6 months under accelerated storage conditions (40 °C and 75% RH). TGA analysis results obtained from Reference example 16 (raw micronized indacaterol maleate, not spray-dried, AN018) and tiotropium bromide monohydrate (not spray-dried, 0006040) are also shown.

TABLE 1: TGA results

| Samples | Accelerated storage conditions (40 °C and 75% RH)<br>Mean (%) ± SD (n=3)<br>Weight loss between 25 °C-125 °C | | | | | |
|---|---|---|---|---|---|---|
| Time point | 0 | 15 days | 1 month | 2 months | 3 months | 6 months |
| Ref. example 16 Indacaterol maleate (AN018) | 0.46±0.08 | 0.34±0.02 | 0.36±0.02 | N.D. | 0.29±0.04 | 0.09±0.03 |
| Tiotropium bromide monohydrate (0006040) | 0.04±0.01 | N.D. | N.D. | N.D. | N.D. | N.D. |
| Ref. example 1 (12J2012) | 1.57±0.03 | 1.67±0.08 | 1.97±0.1 | N.D. | 2.0±0.2 | 3.26 (n=1) |

(continued)

| Samples | Accelerated storage conditions (40 °C and 75% RH)<br>Mean (%) ± SD (n=3)<br>Weight loss between 25 °C-125 °C | | | | | |
|---|---|---|---|---|---|---|
| Time point | 0 | 15 days | 1 month | 2 months | 3 months | 6 months |
| Ref. example 2 (9J2012A) | 4.8±0.2 | 4.97±0.08 | 5.3±0.2 | 3.03±0.04* | 3.08±0.07* | 2.11±0.16* |
| Ref. example 3 (2J2012C) | 4.4±0.1 | 4.11±0.04 | 4.74±0.03 | 1.01±0.09* | 0.94±0.02* | 1.09±0.04* |
| Example 4 (9J2012B) | 3.6±0.1 | 3.50±0.08 | 3.4±0.3 | N.D. | 3.8±0.2 | 3.8±1.0 |
| *recrystallization detected by XRPD, N.D. not determined | | | | | | |

Reference example 2 and Reference example 3 presented instable amorphous form with recrystallization between 1 and 2 months under accelerated storage conditions as confirmed by XRPD and as noticeable from the drastic change of moisture content measured by TGA.

The results presented in Table 2 showed the moisture content determined by % of weight loss between 25°C-140°C of particles of Example 5 (9A2012A), Reference example 6 (9A2012B) at t=0, 15 days, 1 month, 3 months and 6 months under accelerated storage conditions (40°C, 75% RH). TGA analysis results obtained from Reference example 16 (micronized raw indacaterol maleate, not spray-dried, AN018) are also shown.

TABLE 2: TGA results

| Samples | Accelerated storage conditions (40 °C, 75% RH)<br>Mean (%) ± SD (n=3)<br>Weight loss between 25 °C-140 °C | | | | |
|---|---|---|---|---|---|
| Time point | 0 | 15 days | 1 month | 3 months | 6 months |
| Ref. example 16 Indacaterol maleate (AN018) | 0.62 ± 0.08 | N.D. | N.D. | N.D | N.D |
| Example 5 (9A2013A) | 2.7 ± 0.1 | 1.87 ± 0.02 | 1.57 ± 0.05 | 1.6 ± 0.4 | 2.1 ± 0.6 |
| Ref. example 6 (9A2013B) | 3.45 ± 0.09 | 3.2 ± 0.1 | 3.2 ± 0.1 | 3.1 ± 0.3 | 2.2 ± 0.8* |
| *recrystallization detected by XRPD, N.D. not determined | | | | | |

Reference example 6 (9A2013B) presented instable amorphous form with recrystallization between 3 and 6 months under accelerated storage conditions (40°C, 75% RH) as confirmed by XRPD and as noticeable from the drastic change of moisture content measured by TGA.

The results presented in Table 3 showed the moisture content determined by % of weight loss between 25°C-140°C of Reference example 16 (micronized raw indacaterol maleate, not spray-dried , C1148) and particles of Reference example 1 (26C2013A), Example 4 (26C2013B), Example 5 (26C2013C), Reference example 6 (26C2013D) at t=0, 15 days, 1 month, 2 months, 3 months and 6 months under high temperature storage conditions (60°C, 30% RH).

TABLE 3: TGA results

| Samples | High temperature storage conditions (60 °C- 30% RH)<br>Mean (%) ± SD (n=3)<br>Weight loss between 25 °C-140 °C | | | | | |
|---|---|---|---|---|---|---|
| Time point | 0 | 15 days | 1 month | 2 months | 3 months | 6 months |
| Ref. example 16 Indacaterol maleate (C1148) | 0.31 ± 0.03 | 0.04 ± 0.04 | 0.12 ± 0.04 | 0.12 ± 0.06 | 0.04±0.02 | 0.20±0.02 |
| Ref. example 1 (26C2013A) | 1.44 ± 0.05 | 1.6 ± 0.4 | 1.5 ± 0.1 | 1.78 ± 0.03 | 1.8±0.1 | 1.87±0.09 |

(continued)

| Samples | High temperature storage conditions (60 °C- 30% RH)<br>Mean (%) ± SD (n=3)<br>Weight loss between 25 °C-140 °C | | | | | |
|---|---|---|---|---|---|---|
| Time point | 0 | 15 days | 1 month | 2 months | 3 months | 6 months |
| Example 5 (26C2013C) | 1.06 ± 0.07 | 1.4 ± 0.1 | 0.93 ± 0.07 | 1.73 ± 0.30 | 2±1 | N.D |
| Ref. example 6 (26C2013D) | 3.7 ± 0.1 | 0.09 ± 0.02 | 0.07 ± 0.04* | 0.13 ± 0.06* | 0.10±0.03* | 0.10±0.02* |
| *recrystallization detected by XRPD, N.D. not determined | | | | | | |

Reference example 6 (26C2013D) presented instable amorphous form with recrystallization between 15 days and 1 month as confirmed by XRPD and as noticeable from the drastic change of moisture content measured by TGA.

The results presented in Table 4 showed the moisture content determined by % of weight loss between 25 °C-140 °C of different samples at t=0, 1 month, 2 months and 3 months under accelerated storage conditions (40 °C, 75% RH) and under high temperature storage conditions (60 °C, 30% RH).

TABLE 4: TGA results

| 40°C, 75%RH, Mean (%) ± SD (n=3), Weight loss between 25°C-140°C | | | | |
|---|---|---|---|---|
| T= | 0 | 1 month | 2 months | 3 months |
| Ref. example 7 (SI-3A) | 1.7±0.2 | 1.0±0.1 | 0.8±0.2 | 1.1±0.3* |
| Ref. example 8 (ST-3A) | 1.26±0.08 | 0.8±0.2 | / | 0.95±0.06 |
| Example 12 (SITL20-3A) | 1.9±0.5 | 1.2±0.1 | 0.90±0.03 | 1.19±0.09 |
| Example 11 (SITL30-3A) | 2.7±0.5 | 1.7±0.1 | 1.66±0.05 | 1.9±0.3 |
| Example 10 (SITL40-3A) | 2.6±0.3 | 1.2±0.2 | 1.46±0.04 | 1.44±0.08 |
| Example 9 (SITL50-3A) | 2.6±0.1 | 1.6±0.2 | 1.65±0.07 | 1.84±0.12 |
| Example 14 (SIT-3A) | 1.50±0.02 | 0.8±0.2 | / | 0.84±0.05 |
| Ref.example 15 (L99SIT-3A) | 4.59±0.07 | 4.52±0.05 | 4.50±0.08 | 4.60±0.05 |
| Ref.example 17 (RI-A) | <LOD | <LOD | <LOD | <LOD |
| Ref.example 18 (RTMeOH-A) | 16±1 | 7.4±0.8 | 0.3±0.2 | <LOD |
| 60°C, 30%RH<br>Mean (%) ± SD (n=3), Weight loss between 25°C-140°C | | | | |
| T= | 0 | 1 month | 2 months | 3 months |
| Ref. example 7 (SI-3B) | 1.7±0.2 | 0.8±0.2 | 0.93±0.08 | 1.00±0.09 |
| Ref. example 8 (ST-3B) | 1.26±0.08 | 0.68±0.04 | / | 0.65±0.09* |
| Example 12 (SITL20-3B) | 1.9±0.5 | 1.4±0.3 | 1.77±0.08 | 2.02±0.13 |
| Example 11 (SITL30-3B) | 2.7±0.5 | 1.23±0.02 | 1.9±0.1 | 2.1±.4 |
| Example 10 (SITL40-3B) | 2.6±0.3 | 1.1±1 | 1.77±0.08 | 1.62±0.09 |
| Example 9 (SITL50-3B) | 2.6±0.1 | 1.1±0.2 | 1.6±0.3 | 2.1±0.1 |
| Example 14 (SIT-3B) | 1.50±0.02 | 0.63±0.04 | / | 0.92±0.07 |
| Ref. example 15 (L99SIT-3B) | 4.59±0.07 | 4.5±0.2 | 4.5±0.1 | 4.53±0.15 |
| Ref. example 17 (RI-A) | <LOD | <LOD | <LOD | <LOD |

(continued)

| 60°C, 30%RH<br>Mean (%) ± SD (n=3), Weight loss between 25°C-140°C | | | | |
|---|---|---|---|---|
| T= | 0 | 1 month | 2 months | 3 months |
| Ref. example 18 (RTMeOH-B) | 16±1 | <LOD | <LOD | <LOD |
| <LOD because inferior to the detection limit 20 μg/5-10mg i.e. 0.2-0.4%; *crystallization detected by XRPD | | | | |

All inhalable particles presented a moisture content measured by TGA inferior to 5% (from spray-dried solutions and suspensions). All inhalable particles from spray-dried solutions showed moisture content measured by TGA inferior to 3%.

Reference example 7 (SI-A) presented instable amorphous form with recrystallization between 2 and 3 months and Reference example 8 (SIT-B) presented instable amorphous form with recrystallization between 2 and 3 months under high temperature storage conditions (60°C, 30%RH) as confirmed by XRPD. For these products, the recrystallization was not noticeable by TGA as no drastic change of the moisture content was measured by this method.

<u>X-ray Powder Diffraction (XRPD)</u>

XRPD is a powerful and widely-used tool for crystalline state evaluation. Diffraction patterns of formulations were determined using a Siemens Diffractometer D5000 (Siemens, Germany), with a Cu line as the source of radiation (WL1 = 1.5406 A, WL2 = 1.54439 A), and standard runs using a 40 kV voltage, a 40 mA current and a scanning rate of 0.02 °/min over a 2 θ range of 2° to 70°.

XRPD diffractograms of the Reference example 3 (2J2012C), Example 4 (9J2012B), and Example 5 (9A2013A) at T=0 (A) and at T=3 months (B) for the Reference example 3 and Example 5 or t= 6 months (B) for the Example 4 are reported in Figure 1, Figure 2 and Figure 3. The Example 4 and Example 5 presented a stabilized amorphous form whereas the Reference example 3 presented a non-stabilized amorphous form.

XRPD diffractograms of the Reference example 7 (SI-3A), Reference example 8 (ST-3B), Example 11 (SITL30-3A) and Example 10 (SITL40-3A) at T=0 (left) and at T=3 months (right) for the Reference example 7, Reference example 8, Example 11 and Example 10 are reported in Figure 4, Figure 5, Figure 6 and Figure 7. The Example 11 and Example 10 presented a stabilized amorphous form whereas Reference example 7, Reference example 8 presented a non-stabilized amorphous form.
The percentage of crystalline phase in the sample was measured using the surface area ratio method using the Diffractplus EVA software (Bruker

Belgium SA, Brussels, Belgium). The degree of crystallinity was calculated by the formula:

$$\%\ Crystallinity = \left(\frac{A_C}{A_T}\right) \times 100$$

Where, $A_T$ is the total area under the diffractogram and $A_C$ is the area under the diffractogram without integrating the deviation from the baseline. The amorphous content (expressed in %) was estimated as 100% minus the estimated degree of crystallinity.

The results shown in Table 5 show the estimated amorphous content determined by XRPD of Reference example 16 (micronized raw indacaterol maleate, AN018) and particles of, Reference example 2 (9J2012A), Reference example 3 (2J2012C), and Example 4 (9J2012B) at T=0, and after 15 days, 1 month, 2 months, 3 months and 6 months under accelerated storage conditions (40°C, 75% RH).

TABLE 5: XRPD results, percentage of amorphous phase content in the sample

| Storage time at 40 °C, 75%RH | Reference example 16 Indacaterol maleate (AN018) | Reference example 1 (12J2012) | Reference example 2 (9J2012A) | Reference example 3 (2J2012C) | Example 4 (9J2012B) |
|---|---|---|---|---|---|
| T = 0 day | 40% | 100% | 100% | 100% | 100% |
| T = 15 days | 31% | 100% | 100% | 100% | 100% |
| T = 1 month | 36% | 100% | 100% | 100% | 100% |

(continued)

| Storage time at 40 °C, 75%RH | Reference example 16 Indacaterol maleate (AN018) | Reference example 1 (12J2012) | Reference example 2 (9J2012A) | Reference example 3 (2J2012C) | Example 4 (9J2012B) |
|---|---|---|---|---|---|
| T = 2 months | N.D. | 100% | 28% | 50% | 100% |
| T = 3 months | 35% | 100% | 27% | 48% | 100% |
| T = 6 months | 34% | 100% | 27% | 47% | 100% |

Reference example 2 (9J2012A) and Reference example 3 (2J2012C) presented instable amorphous form with recrystallization between 1 and 2 months under accelerated storage conditions (40°C, 75% RH) which are visible from the drastic change of the amorphous content measured by XRPD.

Reference example 16 (AN018) presented a relatively high amorphous content not stable along time under accelerated storage conditions (40°C, 75% RH) which is visible from the slight variations of the amorphous content measured by XRPD.

The results presented in Table 6 show the estimated amorphous content determined by XRPD of particles of Example 5 (9A2013A) and Reference example 6 (9A2013B) at T=0, and after 15 days, 1 month, 2 months, 3 months and 6 months storage under accelerated storage conditions (40 °C, 75% RH).

TABLE 6: XRPD results, percentage of amorphous phase content in the sample

| Storage time at 40°C, 75%RH | Example 5 (9A2013A) | Reference example 6 (9A2013B) |
|---|---|---|
| T = 0 day | 100% | 100% |
| T = 15 days | 100% | 100% |
| T = 1 month | 100% | 100% |
| T = 2 months | 100% | 100% |
| T = 3 months | 100% | 100% |
| T = 6 months | 100% | 35% |

Reference example 6 (9A2013B) presented instable amorphous form with recrystallization between 3 and 6 months under accelerated storage conditions (40°C, 75% RH) which is visible from the drastic change of the amorphous content measured by XRPD whereas Example 5 (9A2013A) presented a stable amorphous form.

The results presented in Table 7 show the estimated amorphous content determined by XRPD of Reference example 16 (micronized raw indacaterol maleate, not spray-dried, C1148) and particles of Reference example 1 (26C2013A), Example 4 (26C2013B), Example 5 (26C2013C), and Reference example 6 (26C2013D) at T=0, and after 15 days, 1 month, 2 months, 3 months and 6 months storage under high temperature storage conditions (60°C, 30% RH).

TABLE 7: XRPD results, percentage of amorphous phase content in the sample

| Storage time at 60 °C, 30%RH | Reference example 16 Indacaterol maleate (C1148) | Reference example 1 (26C2013A) | Example 4 (26C2013B) | Example 5 (26C2013C) | Reference example 6 (26C2013D) |
|---|---|---|---|---|---|
| T = 0 day | 36% | 100% | 100% | 100% | 100% |
| T = 15 days | 31% | 100% | 100% | 100% | 100% |
| T = 1 month | 28% | 100% | 100% | 100% | 40% |
| T = 2 months | 30% | 100% | 100% | 100% | 39% |

Reference example 6 (26C2013D) presented instable amorphous form with recrystallization between 15 days and 1 months, under high temperature storage conditions (60 °C, 30% RH), which are visible from the drastic change of the amorphous content measured by XRPD.

Reference example 16 (C1148) presented a relatively high amorphous content not stable along time under high temperature storage conditions (60 °C, 30% RH) which is visible from the variation of the amorphous content

measured by XRPD. Example 5 presented a 100% of amorphous content at 3 and 6 months under high temperature storage conditions (60 °C, 30% RH) (data not shown in the table above).

The results presented in Table 8 showed the estimated amorphous content determined by XRPD of different samples at T=0, and after 1 month, 2 and 3 months storage under accelerated storage conditions (40 °C, 75% RH) and under high temperature storage conditions (60 °C, 30% RH).

TABLE 8: XRPD results, percentage of amorphous phase content in the sample

| Amorphous content (%) 40°C, 75%RH | | | | |
|---|---|---|---|---|
| T = | T0 | 1 month | 2 months | 3 months |
| Ref. example 7 (SI-3A) | 100% | 100% | 100% | 94% |
| Example 12 (SITL20-3A) | 100% | 100% | 100% | 100% |
| Example 11 (SITL30-3A) | 100% | 100% | 100% | 100% |
| Example 10 (SITL40-3A) | 100% | 100% | 100% | 100% |
| Example 9 (SITL50-3A) | 100% | 100% | 100% | 100% |
| Example 14 (SIT-3A) | 100% | 100% | / | 100% |
| Ref. example 15 (L99SIT-3A) | 20% | 20% | 22% | 20% |
| Ref. example 17 (RI-A) | 13% | 16% | 19% | 16% |
| Ref. example 18 (RTMeOH-A) | 32% | 43% | 46% | 37% |
| Amorphous content (%) 60 °C, 30%RH | | | | |
| T = | 0 | 1 month | 2 month | 3 months |
| Ref. example 8 (ST-3B) | 100% | 100% | N.D. | 81% |
| Example 12 (SITL20-3B) | 100% | 100% | 100% | 100% |
| Example 11 (SITL30-3B) | 100% | 100% | 100% | 100% |
| Example 10 (SITL40-3B) | 100% | 100% | 100% | 100% |
| Example 9 (SITL50-3B) | 100% | 100% | 100% | 100% |
| Example 14 (SIT-3B) | 100% | 100% | N.D. | 100% |
| Ref. example 15 (L99SIT-3B) | 20% | 18% | 20% | 19% |
| Ref. example 17 (RI-A) | 13% | 17% | 16% | 16% |
| Ref. example 18 (RTMeOH-B) | 32% | 34% | 36% | 30% |

Reference example 17 (RI-A and RI-B) presented variations in amorphous form under both storage conditions, which are visible from the change of amorphous content measured by XRPD. Reference example 18 (RTMeOH-A and RTMeOH-B) presented variations in amorphous form under both storage conditions, which are visible from the change of amorphous content measured by XRPD.

Reference example 7 (SI-3A) presented instable amorphous form with recrystallization between 2 and 3 months under accelerated storage conditions (40 °C, 75%RH), which are visible from the change of amorphous content measured by XRPD. Reference example 8 (ST-3B) presented instable amorphous form with recrystallization between 2 and 3 months under high temperature storage conditions (60 °C, 30% RH), which are visible from the change of amorphous content measured by XRPD..

Example 12 (SITL20-3A, SITL20-3B), Example 11 (SITL30-3A, SITL30-3B), Example 10 (SITL40-3A, SITL40-3B), Example 9 (SITL50-3A, SITL50-3B) and Example 14 (SIT-3A, SIT-3B) presented stable amorphous forms without recrystallization after 3 months in both storage conditions (40 °C, 75% RH and 60 °C, 30% RH).

Scanning electron microscopy (SEM)

Evaluation of particle size and morphology was achieved by Scanning Electron Microscopy (SEM), using a SU-70 (Hitachi, Japan) or a JSM-6100 microscope (Jeol, Japan). Samples were scattered on a thin film of a two-component epoxy resin and then coated with a platinum layer. Acceleration during observation was 25 kV.

SEM images of Reference example 3 (2J2012C), Example 4 (9J2012B) and Example 5 (9A2013A) at T=0 (left) and T = 3 months (right) for Reference example 3 and Example 5 or T= 6 months (right) for Example 4 are shown in Figure 8, Figure 9 and Figure 10.

SEM images of Example 11 (SITL30-3A) and Example 10 (SITL40-3A) at T=0 (left) and T = 2 months (right) are shown in Figure 11 and Figure 12.

Inhalable particles obtained after spray-drying presented a spherical shape. In the sample presenting recrystallization (Reference example 3), crystals presenting non-spherical shape and bigger size were observed in SEM pictures whereas the stable particles preserved the initial shape and size (Example 4, Example 5, Example 11 and Example 10).

Hot stage microscopy (HSM)

HSM analysis was conducted using a Linkam THMS 600 hot stage (England) assembled on an Olympus-BX 60 microscope (Japan), equipped with a JVC TK-C1381 (Japan) color video camera. The samples were observed under the microscope by using polarized and non-polarized light in order to determine their physical state (amorphous-crystalline).

At T=0, samples of Reference example 3 (2J2012C), Example 4 (9J2012B) and Example 5 (9A2013A) showed no crystalline particles (data not shown) which confirmed the amorphous state of all components. After 3 months, crystallization was observed for the sample of Reference example 3 but not for the sample of Example 4 and Example 5. The samples of Example 4 and Example 5 did not show crystallization after 6 months either. These results confirmed the results obtained by XRPD and TGA analysis.

As described before, drug particles in amorphous state are not stable and may show physical transformation (amorphous to crystalline state) during storage. It has been shown that amorphous state can be stabilized in certain conditions using an appropriate stabilizing agent.

Amorphous indacaterol (Reference example 7), amorphous tiotropium (Reference example 8) and amorphous indacaterol-lactose (Reference examples 2 and 6) or indacaterol-tiotropium-lactose with a content of lactose higher than 85% (Reference examples 3) showed recrystallization during storage at accelerated and/or high storage conditions (40 °C, 75% RH and 60 °C, 30% RH). Surprisingly, the combination of two active ingredients (i.e. indacaterol and tiotropium) without lactose (Examples 5 and 14) or the combination of indacaterol and tiotropium with 20, 30, 40, 50 and 55.6% lactose (Examples 4, 9-12) both in an intimate mixture within a matrix (ingredients dispersed at molecular level) obtained by using an appropriate drying method from solutions, permitted to stabilize both drugs in amorphous state.

Evaluation of the chemical stability (drug degradation)

High Performance Liquid Chromatography with UV detection (HPLC-UV) The HPLC system consisted of a High Performance Liquid Chromatography system (HP 1100 series, Agilent technologies), equipped with a single pump, an autosampler and a UV detector. The following conditions were adopted: the column was a 125 mm x 4 mm Purospher® STAR RP-18 endcapped (5 µm) (Merck N° 1.50036.0001), the mobile phase was, for example, a mixture of phosphate buffer pH 4 (13.6 g/L of $KH_2PO_4$) and Acetonitrile (ACN) (70/30 v/v) filtered on Millipore 0.22 µm GV (Cat. GVWP02500), the dilution solvent was, for example, MilliQ water/ACN (70/30 v/v). The chromatographic conditions were: a flow rate of 0.8 mL/min, a temperature set at 35 °C, an injection volume of 100 µL and a typical run time of 10 minutes. The wavelength was set, for example, at 238 nm (for tiotropium) and 260 nm (for indacaterol)The standard and samples were filtered on ProFill, 25mm HPLC Syringe filter 0.45 µm (Grace Cat. N°: 25 16 0349).

Alternatively, for the determination of indacaterol in samples of Table 9 and 11, the HPLC system consisted of a High Performance Liquid Chromatography system (HP 1100 series, Agilent technologies), equipped with a single pump, an autosampler and a UV detector. The following conditions were adopted: the column was a 125 mm x 4 mm Purospher® STAR RP-18 endcapped (5 µm) (Merck N° 1.50036.0001), the mobile phase was, for example, a mixture of phosphate buffer pH 4 (13.6 g/L of $KH_2PO_4$) and Acetonitrile (ACN) (70/30 v/v) filtered on Millipore 0.22 µm GV (Cat. GVWP02500), the dilution solvent was, for example, MilliQ water/ACN (70/30 v/v). The chromatographic conditions were: a flow rate of 0.8 mL/min, a temperature set at 35 °C, an injection volume of 100 µL and a typical run time of 10 minutes. The wavelength was set, for example, 260 nm (for indacaterol). The standard and samples were filtered on ProFill, 25mm HPLC Syringe filter 0.45 µm (Grace Cat. N°: 25 16 0349).

For the determination of tiotropium and indacaterol of the samples of Table 12, the HPLC system consisted of a High Performance Liquid Chromatography system (HP 1200 series, Agilent technologies), equipped with a single pump, an autosampler and a DAD detector. The mobile phase was, for example, a mixture of phosphate buffer pH 4 (13.6 g/L of $KH_2PO_4$) and Acetonitrile (ACN) (80/20 v/v) filtered on Millipore 0.22 µm GV (Cat. GVWP02500), and the dilution solvent was, for example, MilliQ water/ACN (80/20 v/v). The chromatographic conditions were: a flow rate of 0.8

mL/min, a temperature set at 35 °C, an injection volume of 100 μL and a typical run time of 60 minutes. The wavelength was set, for example, at 238 nm (for tiotropium) and 260 nm (for indacaterol).

Standards Preparation

For example, about 32.39 mg of indacaterol maleate reference and about 25.00 mg of tiotropium bromide monohydrate were exactly weighted and solubilized in 100-mL flask with dilution phase to constitute the solution at 250 μg/mL of indacaterol base and at 200 μg/ml of tiotropium base. Then, this solution was diluted to elaborate a solution at 10 μg/mL of indacaterol base and at 8 μg/mL of tiotropium base. This solution at 10 μg/mL of indacaterol base and at 8 μg/mL of tiotropium base was used to elaborate the standard solutions at 0.05 μg/mL of indacaterol base and 0.04 μg/mL of triotropium base, 0.1 μg/mL of indacaterol base and 0.08 μg/mL of triotropium base, 0.2 μg/mL of indacaterol base and 0.16 μg/mL of triotropium base, 0.5 μg/mL of indacaterol base and 0.4 μg/mL of triotropium base, 1 μg/mL of indacaterol base and 0.8 μg/mL of triotropium base and 2 μg/mL of indacaterol base and 1.6 μg/mL of triotropium base.

For the method determining indacaterol only, about 32.39 mg of indacaterol maleate reference was exactly weighted and solubilized in 100-mL flask with dilution phase to constitute the solution at 250 μg/mL of indacaterol base. Then, this solution was diluted to elaborate a solution at 10 μg/mL of indacaterol base. This solution at 10 μg/mL of indacaterol base was used to elaborate the standard solutions at 0.05 μg/mL of indacaterol base, 0.1 μg/mL of indacaterol base, 0.2 μg/mL of indacaterol base, 0.5 μg/mL of indacaterol base, 1 μg/mL of indacaterol base and 2 μg/mL of indacaterol base.

For the method determining both, indacaterol and tiotropium, about 19.44 mg of indacaterol maleate reference and about 22.50 mg of tiotropium bromide monohydrate were exactly weighted and solubilized in a 200-mL flask with dilution phase to constitute a solution at 75 μg/mL of indacaterol base and at 90 μg/ml of tiotropium base. Then, this solution was diluted to elaborate:

- a standard solution at 1.875 μg/mL of indacaterol base and at 2.251 μg/mL of tiotropium base,
- a standard solution at 1.500 μg/mL of indacaterol base and at 1.801 μg/mL of tiotropium base,
- a standard solutions at 1.125 μg/mL of indacaterol base and 1.351 μg/mL of tiotropium base,
- a standard solution at 0.750 μg/mL of indacaterol base and 0.900 μg/mL of tiotropium base,
- a standard solution at 0.375 μg/mL of indacaterol base and 0.450 μg/mL of tiotropium base,

The standard solution at 1.500 μg/mL of indacaterol base and 1.801 μg/mL of tiotropium base was used to elaborate a standard solution at:

- 0.150 μg/mL of indacaterol base and 0.1801 μg/mL of tiotropium base The standard solution at 0.750 μg/mL of indacaterol base and 0.9 μg/mL of tiotropium base was used to elaborate the standard solution at:
- 0.075 μg/mL of indacaterol base and 0.090 μg/mL of tiotropium base

Drug content determination

10.00 mg of powder samples were exactly weighted and solubilized in 100-mL flask with dilution phase. Then an appropriate dilution was performed to be in the linear calibration curve.

For Reference example 7 (SI-3A, SI-3B), Reference example 8 (ST-3A, ST-3B), Reference example 17 (RI-A, RI-B) and Reference example 18 (RTMeOH-A, RTMeOH-B), about 20.00 mg of powder samples were exactly weighted and solubilized in a 100-mL flask with the dilution phase. Then appropriate dilution was performed to be in the linear calibration curve (dilution factor 100).

For Example 14 (SIT-3A, SIT-3B), Example 12 (SITL20-3A, SITL20-3B), Example 11 (SITL30-3A, SITL30-3B), Example 10 (SITL40-3A, SITL40-3B) and Example 9 (SITL50-3A, SITL50-3B), about 20.00 mg of powder samples were exactly weighted and solubilized in a 100-mL flask with the dilution phase. Then appropriate dilution was performed to be in the linear calibration curve (dilution factor 100 to determine indacaterol base and dilution factor 10 to determine tiotropium base).

Powder appearance evolution and drug content

Table 9 shows the powder appearance of Reference example 16 (micronized raw indacaterol maleate, not spray-dried, AN018), and spray-dried powders of Reference examples 1-3 and Example 4 after 6 months under accelerated storage stability conditions (i.e. 40 °C-75% RH).

TABLE 9

| 40 °C-75% RH | 6 months | | | | |
|---|---|---|---|---|---|
| | Fine powder | Aggregates | Coloration | Recrystallization | % indacaterol relative to theoretical drug content (n=3) |
| Ref. example 16 Indacaterol maleate (AN018) | Yes | Yes but in lower extent | Off-white | Yes | 96.0 ± 0.6 |
| Ref. example 1 (12J2012) | Yes | Yes | Brown | No | 90 ± 1% |
| Ref. example 3 (2J2012C) | Yes | Yes | Beige | Yes | 95 ± 1% |
| Ref. example 2 (9J2012A) | Yes | No | Off-white | Yes | 94 ± 1% |
| Example 4 (9J2012B) | Yes | Yes | Off-white | No | 89.6 ± 0.3% |

Table 10 shows the powder appearance of the spray-dried powders of Example 5 and Reference Example 6 after 3 months under accelerated storage stability conditions (i.e. 40 °C-75% RH).

TABLE 10

| 40 °C-75%RH | 3 months | | | |
|---|---|---|---|---|
| | Fine powder | Aggregates | Coloration | Recrystallization |
| Example 5 (9A2013A) | Yes | Yes | Off-white | No |
| Ref. example 6 (9A2013B) | Yes | Yes | White | No |

Table 11 shows the powder appearance of Reference example 16 (micronized raw indacaterol maleate, not spray-dried, C1148), and the spray-dried powders of Reference example 1, Example 4, Example 5, and Reference Example 6 under high temperature storage conditions (i.e. 60 °C - 30% RH) during 1 month. The drug content was evaluated after 6 weeks.

TABLE 11

| 60 °C-30% RH | 1 month | | | | 6 weeks |
|---|---|---|---|---|---|
| | Fine powder | Aggregates | Coloration | Recrystallization | % indacaterol relative to theoretical drug content (n=3) |
| Ref. example 16 Indacaterol maleate (C1148) | Yes | Yes | Off-white but in lesser extent | Yes | 97.9 ± 0.8% |
| Ref. example 1 (26C2013A) | Yes | Yes | Off-white | No | 89 ± 1% |
| Example 4 (26C2013B) | Yes | Yes | Off-white | No | 84.9 ± 0.7% |
| Example 5 (26C2013C) | Yes | Yes | Off-white | No | 90 ± 4% |
| Ref. example 6 (26C2013D) | Yes | Yes | Off-White | Yes | 94.6 ± 0.3% |

Although crystalline indacaterol maleate was more resistant under accelerated storage conditions (40 °C - 75%

RH) and under high temperature storage conditions (60 °C - 30% RH), the degradation for particles staying amorphous during 6 months under accelerated storage conditions (10% of degradation in 6 months) or during 6 weeks under high temperature storage conditions was relatively limited (10-15% of degradation).

Table 12 shows the percentage of indacaterol and tiotropium in Reference examples 7, 8, 17 and 18, and Examples 9-12 and 14 at T=0, 1 and 2 months expressed in relation to the drug content at T=0 under accelerated storage conditions (i.e. 40 °C - 75% RH), determined by HPLC-UV.

TABLE 12

| | Accelerated storage conditions (40°C, 75%RH) | | | | | | | |
|---|---|---|---|---|---|---|---|---|
| | Indacaterol | | | | Tiotropium | | | |
| T (month) | 0 | 1 | 2 | 4 | 0 | 1 | 2 | 4 |
| Ref. example 7 (SI-3A) | 100±2 | 101±1 | 95.6±0.3 | 95±2 | | | | |
| Ref. example 8 (ST-3A) | | | | | 100.0±0.9 | 99.7±0.6 | N.D. | 94±5 |
| Example 12 (SITL20-3A) | 100±1 | 98±3 | 93±4 | N.D. | 100±1 | 99±3 | 103±1 | N.D. |
| Example 11 (SITL30-3A) | 100±2 | 98±2 | 101±3 | 103±1 | 100.0±0.4 | 100±1 | 102±1 | 105±3 |
| Example 10 (SITL40-3A) | 100±1 | 96±3 | 95±2* | N.D. | 100.0±0.7 | 96±4 | 102±1 | N.D |
| Example 9 (SITL50-3A) | 100. 0±0.8 | 94±6 | 97±1* | N.D. | 100±1 | 95±2 | 100±3 | N.D. |
| Example 14 (SIT-3A) | 100±1 | 95±4 | N.D. | 97±2 | 100.0±0.8 | 101±2 | N.D. | 110±2 |
| Ref. example 17 (RI-A) | 100±2 | 103±1 | 97±3 | 101±1 | | | | |
| Ref. example 18 (RTMeOH-A) | | | | | 100.0±0.9 | 100±1 | 95.5±0.1 | 100.1±0.7 |
| n=3, *n=2; #crystallization detected by XRPD | | | | | | | | |

Table 13 shows the percentage of indacaterol and tiotropium in Reference examples 7, 8, 17 and 18, and Examples 9-12 and 14 at T=0, 1, 2 and 4 months expressed in relation to the drug content at T=0 under high temperature storage conditions (i.e. 60 °C - 30% RH), determined by HPLC-UV.

TABLE 13

| | Accelerated storage conditions (60°C, 30%RH) | | | | | | | |
|---|---|---|---|---|---|---|---|---|
| | Indacaterol | | | | Tiotropium | | | |
| T(month) | 0 | 1 | 2 | 4 | 0 | 1 | 2 | 4 |
| Ref. example 7 (SI-3B) | 100±2 | 99±1 | 89±3 | 92±2 | | | | |
| Ref. example 8 (ST-3B) | | | | | 100.0±0.9 | 99.7±0.6 | N.D. | 100.1± 0.4 |
| Example 12 (SITL20-3B) | 100±1 | 97±3 | 94.7±0.7 | N.D. | 100±1 | 101±1 | 99.2±0.3 | N.D. |
| Example 11 (SITL30-3B) | 100±2 | 99±4 | 92.3±0.3 | 87±2 | 100.0±0.4 | 101±1 | 101.5±0.5 | 98±2 |
| Example 10 (SITL40-3B) | 100±1 | 94.9±0.1 | 86±3 | N.D. | 100.0±0.7 | 96±1 | 95±3 | N.D. |
| Example 9 (SITL50-3B) | 100.0±0. 8 | 97±2 | 87±3 | N.D. | 100±1 | 96±1 | 94±3 | N.D. |
| Example 14 (SIT-3B) | 100±1 | 101±4 | 93±1 | 85±6 | 100.0±0.8 | 100.9±0.9 | 105±1 | 108±2 |
| Ref. example 17 (RI-A) | 100 ±2 | 97.8±0.4 | 97±3 | 99±6 | | | | |
| Ref. example 18 (RTMeOH-B) | | | | | 100.0±0.9 | 98.4±0.6 | 98±1 | 100±6 |
| *n=2 (n=3); #crystallization detected by XRPD | | | | | | | | |

Although crystalline indacaterol maleate was more resistant under accelerated storage conditions (40 °C - 75%RH) and under high temperature storage conditions (60 °C - 30%RH), the degradation for particles staying amorphous during 2 months under accelerated storage conditions (10% of degradation in 2 months) or under high temperature storage conditions was shown to be relatively limited (10-15% of degradation).

Evaluation of the pharmaceutical properties (uniformity of drug content and of the delivered dose, aerodynamic performances, drug dissolution)

Uniformity of drug content

Uniformity of drug content was assessed using the test B of uniformity of content of single-dose preparations of European Pharmacopeia 7.0, which consists to determine the individual contents of active substance(s) in 10 dosage units (about 25 ± 2 mg exactly weighted in a No. 3 HPMC capsule). The same procedure was performed for the Onbrez® for the indacaterol content and Spiriva® for the tiotropium content.

Each capsule was placed in 20-mL flask and filled partly with dilution phase. The flask was then placed in an ultrasonic bath until the complete dissolution of the capsule and the formulation. Then, the volume was made up to the mark when the solution was at room temperature. Further dilution could be required to obtain the concentration of the compound in the linearity range of the HPLC-UV method of determination. Typically, no dilution was performed to determine tiotropium and a dilution of 10 was applied to determine indacaterol.

Example 21 and Example 22 as well as the reference comparator products (Onbrez® and Spiriva®) complied with the test of uniformity of content of single-dose preparations (Test B) of European Pharmacopeia 7.0, tested on 10 dosage units as no more than one individual content was outside the limits of 85% to 115% of the average content and none was outside the limits of 75% to 125% of the average content. Table 14 shows the average value (mean) ± the standard deviation (SD) and the coefficient of variation (CV %). The results obtained were similar both in terms of drug content and drug content homogeneity for the formulations of the invention and the reference comparator products.

Uniformity of delivered doses

The uniformity of the delivered dose (UDD) was measured using a UDD device (Copley Scientific Ltd., Nottingham, UK) and the procedure described in European Pharmacopoeia 7.0 for assessment of powders for inhalation. The filter used was a Glass fiber Filter Millipore AP4004700 (47 mm diameter).For the Indacaterol-tiotropium DPI formulations of the invention (Example 21 and Example 22): a Fantasmino® inhaler was filled with a N°3 HPMC capsule loaded with 25 ± 2 mg of the formulation. The flow rate was set to 100 L/min for 2.4 seconds to obtain a pressure drop of 4 kPa across the device as recommended by European Pharmacopeia 7.0.

After each test, the glass filter was placed in 50-mL flask and the UDD device was rinsed several times with the dilution phase. The flask was then placed in ultrasonic bath until the complete dissolution of the filter and the powder. Then, the volume was made up to the mark when the solution was at room temperature. Further dilution could be required to obtain the concentration of the compound in the linearity range of the HPLC-UV method of determination. Typically, no dilution was performed to determine tiotropium and a dilution of 5 was applied to determine indacaterol.

Example 21 and Example 22 complied with the test of uniformity of delivered dose at 100 L/min during 2.4 seconds through Fantasmino® inhaler as at least 9 out 10 results lie between 75% and 125% of the average delivered dose value and all values lie between 65% and 135%. Table 14 shows the average values (mean) ± the standard deviation (SD) and the minimum and maximum values ($\mu g_{min}$-$\mu g_{max}$). The delivered dose of the reference products (Spiriva® and Onbrez®) was reported from the product monographs provided by the suppliers. Table 15 showed the same delivered dose data but related to the drug content. Example 21 presented mean delivered doses of 65% (range 58-72%) of indacaterol and 72% (range 64-84%) of tiotropium, and Example 22 presented delivered doses of 64% (range 53-70%) of indacaterol and 69% (range 58-80%) of tiotropium in relation to the nominal doses of indacaterol and tiotropium, which were emitted through the Fantasmino® inhaler. Whereas the reference products presented different percentages of emission, 79% of indacaterol from Onbrez® Breezhaler® and 56% of tiotropium from Spiriva® Handihaler®, Example 21 and 22 showed thus a closer emission of both drugs using one single device.

Aerodynamic particle-size analysis

The aerodynamic particle size distribution for the indacaterol-tiotropium DPI formulations (Example 21 and Example 22) and the different reference comparator products (Onbrez® Breezhaler® and Spiriva® Handihaler®) was determined using a fast screening impactor (FSI).

The Fantasmino® inhaler was used with a No. 3 HPMC capsule for indacaterol-tiotropium DPI formulations (Example 21 and Example 22). The flow rate was set to 100 L/min for 2.4 seconds to obtain a pressure drop of 4 kPa across the

device as recommended by European Pharmacopeia 7.0. During the FSI experiments, the dose recovered for each run was in the range of 75-125% of the nominal dose. Example 21 and Example 22 presented a co-deposition of indacaterol and tiotropium in each part of the impactor as reported in Table 15. Example 21 presented about 27-29% of indacaterol and tiotropium staying in the device, about 7-8% of indacaterol and tiotropium deposited in the induction port, about 40-43% of indacaterol and tiotropium deposited in the pre-separator and about 22-25% indacaterol and tiotropium deposited in the filter (particles presenting an aerodynamic diameter inferior to 5 μm). Example 22 presented about 21-22% of indacaterol and tiotropium staying in the device, about 18.3-18.9% of indacaterol and tiotropium deposited in the induction port, about 32-33% of indacaterol and tiotropium deposited in the pre-separator and about 27.6-28.2% indacaterol and tiotropium deposited in the filter.

The reference comparator products (Onbrez® Breezhaler® and Spiriva® Handihaler®) presented completely different pattern of deposition in each part, 13.9% of indacaterol or 40% of tiotropium in the device; 17% of indacaterol or 9% of tiotropium in the induction port; 25% of indacaterol or 16% of tiotropium in the pre-separator, and 44.4% of indacaterol or 35% of tiotropium in the filter.

Table 14 shows the results of uniformity of drug content (μg; mean ± SD (CV %), n=3), uniformity of delivered dose (μg; mean ± SD ($\mu g_{min}$-$\mu g_{max}$), n=3), aerodynamic particle-size analysis by FSI (μg/capsule; mean ± SD, n=3) for the reference comparator products Onbrez® and Spiriva® and the indacaterol-tiotropium formulations of the invention of Example 21 and Example 22.

TABLE 14

| | | | FSI (mean ± SD, n = 3), μg/capsule | | | |
|---|---|---|---|---|---|---|
| | Drug content (μg) mean ± SD (CV%) | Delivered dose (μg) mean ± SD ($\mu g_{min}$-$\mu g_{max}$) | Device | Ind, Port | Pre-separator | Filter = FPD |
| Onbrez® Indacaterol | 152 ± 3 (2%) | 120* | 17.2 ± 0.4 | 21 ± 4 | 31 ± 2 | 55 ± 2 |
| Spiriva® Tiotropium | 18 ± 1 (3%) | 10* | 6.6 ± 0.6 | 1.5 ± 0.1 | 2.5 ± 0.1 | 5.7 ± 0.4 |
| Example 21 24G2013A Indacaterol | 149 ± 3 (2%) | 97 ± 8 (87-108) | 34.8 ± 0.7 | 8 ± 2 | 54 ± 2 | 29 ± 6 |
| Example 21 24G2013A Tiotropium | 17 ± 1 (4%) | 12 ± 1 (11-15) | 4.4 ± 0.2 | 1.3 ± 0.1 | 6.7 ± 0.3 | 4.1 ± 0.5 |
| Example 22 24G2013B Indacaterol | 148 ± 8 (6%) | 95 ± 7 (79-104) | 28 ± 1 | 24.1 ± 0.6 | 42.9 ± 0.3 | 36.1 ± 0.6 |
| Example 22 24G2013B Tiotropium | 18 ± 1 (6%) | 12 ± 1 (10-14) | 3.7 ± 0.1 | 3.1 ± 0.1 | 5.49 ± 0.04 | 4.80 ± 0.08 |
| *Data reported from the product monographs of Onbrez® Breezhaler® and Spiriva® Handihaler® | | | | | | |

Table 15 shows the results of uniformity of drug content (μg; mean ± SD (CV%), n=3), uniformity of delivered dose (% related to nominal dose; mean ± SD ($\%_{min}$-$\%_{max}$), n=3), aerodynamic particle-size analysis by FSI (% related to recovered dose; mean ± SD, n=3) for the reference comparator products Onbrez® and Spiriva® and the indacaterol-tiotropium formulations of Example 21 and Example 22).

TABLE 15

| | | | FSI (mean ± SD, n = 3), % in relation to the recovered dose | | | |
|---|---|---|---|---|---|---|
| | Drug content mean ± SD (CV%) | Delivered dose mean ± SD ($\%_{min}$-$\%_{max}$) | Device | Ind, Port | Pre-separator | FPF recovered |
| Onbrez® Indacaterol | 152 ± 3 (2%) | 79% | 13.9±0.5% | 17±2% | 25±2% | 44.4±0.4% |
| Spiriva® Tiotropium | 18 ± 1 (3%) | 56% | 40±2% | 9±1% | 16±1% | 35±2% |
| Example 21 24G2013A Indacaterol | 149 ± 3 (2%) | 65±5% (58-72) | 28±1% | 7±1% | 42±1% | 22±5% |
| Example 21 24G2013A Tiotropium | 17.4 ± 0.7 (4%) | 72±7% (64-84) | 27±2% | 7.9±0.5% | 40±2% | 25±3% |
| Example 22 24G2013B Indacaterol | 148 ± 8 (6%) | 64±5% (53-70) | 21±1% | 18.4±0.4 % | 32.8±0.3 % | 27.6±0.5% |
| Example 22 24G2013B tiotropium | 18 ± 1 (6%) | 69±6% (58-80) | 21.8±0.8 | 18.3±0.6 % | 32.0±0.1 % | 27.9± 0.3% |

Evaluation of drug release from the dry powder formulations

Dissolution tests using an FSI-based cassette were performed to determine the drug release/dissolution. The evaluation of the dissolution properties of the dry powders were assessed using a dissolution vessel with a paddle apparatus (USP 33 type 2 apparatus). The vessels were filled with 350 ml (FSI-based cassette) of phosphate buffer saline pH 7.4 with 0.1% Tween 80 (m/v). The composition of the phosphate buffer medium was 32 g NaCl, 0.8 g KCl, 5.76 g $Na_2HPO_4$, 0.96g $KH_2PO_4$ and water ad 4 L, and pH adjusted to 7.4 with NaOH 1N or HCl 1N. The paddle speed was fixed at 50 rpm and the temperature was set and maintained at 37.0 °C throughout the tests. Indacaterol and tiotropium were quantified at pre-determined intervals (5, 10, 20, 60, 120 and 180 min) with a suitable HPLC method. In this purpose, 2 ml of dissolution media was removed from the dissolution vessel at different time intervals and directly replaced with 2 ml of fresh dissolution medium. These 2 mL were directly filtered through 0.45 μm diameter filters to avoid quantification of undissolved particles.

A Fast Screening Impactor, FSI (Copley Scientific Limited, Nottingham, UK) using a two separation stage process, equipped with an induction port and a pre-separator was used to determine the dissolution of dry powder particles presenting an aerodynamic diameter inferior to 5 μm. The FSI was used with an adapted insert (Copley Scientific Limited, 40, 60 or 100 L/min) generating a 5 μm cut-off diameter at a flow rate of 40, 60 or 100 L/min. A 90 mm hydrophobic PTFE membrane filter, filter type 0.45μm FH (Fluoropore Cat N°FHLP09050, Millipore, Belgium) was placed in the lower collection plate of the FSI, such that the fine particles (< 5 μm) were captured directly on the membrane.

After the dose actuation step, the membrane was sandwiched between the glass and PTFE surfaces of the watchglass/PTFE assembly (Copley Scientific Limited, Nottingham, UK) with the powder exposed toward to the medium. The watchglass/PTFE assembly was then placed in the bottom of a dissolution vessel (USP 33 type 2 apparatus) with a paddle apparatus, and dissolution testing was conducted as describe above. Each test was replicated three times.

The cumulative amount of drug release was calculated and expressed as the percentage of the cumulative amount of drug release at the end of the dissolution test (i.e., 180 min) and plotted versus time. In this case, the flow rate used allowed the collection of particles presenting an aerodynamic diameter inferior to 5 μm.

For Onbrez® Breezhaler®: 10 Onbrez® capsules containing 150 μg of indacaterol in the Breezhaler® inhaler were used for each test to obtain about 540 μg of indacaterol on the filter. The flow rate was set to 100 L/min for 2.4 seconds to obtain a total air volume of 4L and a pressure drop of 4 kPa across the device as recommended by European Pharmacopeia 7.0.

For Spiriva® Handihaler®: 10 Spiriva® capsules containing 18 μg of tiotropium in the Handihaler® inhaler were used for each test to obtain about 57.6 μg of tiotropium on the filter. The flow rate was set to 40 L/min for 6 seconds to

obtain a total air volume of 4L and a pressure drop of 4 kPa across the device as recommended by European Pharmacopeia 7.0.

For Example 21 (indacaterol-tiotropium DPI formulation, 24G2013A): 18 No. 3 HPMC capsules filled with about 25 ± 2 mg of indacaterol-tiotropium DPI formulation in the Fantasmino inhaler were used for each test to obtain about 540 μg of indacaterol and about 57.6 μg of tiotropium on the filter. The flow rate was set to 100 L/min for 2.4 seconds to obtain a total air volume of 4L and a pressure drop of 4 kPa across the device as recommended by European Pharmacopeia 7.0.

For Example 22 (indacaterol-tiotropium DPI formulation, 24G2013B): 15 No. 3 HPMC capsules filled with about 25 ± 2 mg of indacaterol-tiotropium DPI formulation in the Fantasmino inhaler were used for each test to obtain about 540 μg of indacaterol and about 57.6 μg of tiotropium on the filter. The flow rate was set to 100 L/min for 2.4 seconds to obtain a total air volume of 4L and a pressure drop of 4 kPa across the device as recommended by European Pharmacopeia 7.0.

Dissolution profiles with indacaterol release from the reference comparator product Onbrez®, from Example 21 (24G2013A), and from Example 22 (24G2013B) and with the tiotropium release from the reference comparator product Spiriva®, from Example 21 (24G2013A), and from Example 22 (24G2013B) are shown in Figure 13 and Figure 14.

Example 21 presented a faster release of the poorly water soluble indacaterol in comparison with the reference product Onbrez®, so a closer dissolution profile between indacaterol and tiotropium is observed for this formulation, whereas Example 22 presented a similar release of indacaterol in comparison with the reference product Onbrez®. The highly water soluble tiotropium presented a fast release from Example 21, Example 22 and from the reference product Spiriva®.

It is well known that obtaining homogeneous mixtures for blends based on the presence micronized drugs used at low dose and coarse carriers is a critical operation and highly dependent on the physico-chemical properties of the contained ingredients (i.e. size, shape, density, surface properties). This is also the case for the performances of the dry powder formulations such as the delivered doses or the aerodynamic performances. Moreover, for dry powder formulations based on the combination of two drugs (e.g. indacaterol and tiotropium), the ratios of the delivered doses from the inhaler and the ratios of deposited doses in the different parts of the lung for the drugs can show some variabilities depending on the physico-chemical properties of the contained ingredients. Moreover, batch to batch variabilities can also be observed from small variations of the physical properties of the micronized drugs and the coarse carrier.

As shown in the several examples used to illustrate the performances of the dry powder formulations issued from this invention, the amounts of indacaterol and tiotropium (expressed in percentage of the recovered dose) delivered from the device (UDD) or deposited in the different part of the impactor (device, induction port, pre-separator, FPF) were comparable. It means that for the examples of the invention, the ratios between the delivered doses or the deposited doses of indacaterol and tiotropium were comparable. One can therefore increase the efficacy of the treatment by simply modifying the nominal doses of indacaterol and tiotropium in the dry powder matrix formulation in order to obtain optimal drug doses in the lung and optimal synergic effect for the drugs.

Finally, the drug dissolution from conventional dry powder formulations for inhalation shows a fast dissolution rate for a highly hydrophilic and hydrosoluble drug (e.g. tiotropium bromide), whereas the dissolution is much more slower for a hydrophobic and poorly water-soluble drug (e.g. indacaterol). Again, the examples of the invention have shown that by combining indacaterol and tiotropium into an amorphous intimate mixture, some control of drug release can be obtained as close dissolution profiles between indacaterol and tiotropium can be obtained. One can therefore increase the efficacy of the treatment by controlling the rate of dissolution of each drug from the formulation in order to obtain optimal pharmacological activity in the site of action.

REFERENCES CITED IN THE APPLICATION

US5610163
US6878721
US2004/0132759
WO2012/106575


## Claims

1. Inhalable particles consisting of an intimate mixture which consists of:

    a) an amorphous tiotropium compound,

b) an amorphous indacaterol compound, and
c) a sugar derivative,

wherein the weight of the sugar derivative is comprised from 0 to 85% relative to the weight of the inhalable particles.

2. The inhalable particles according to claim 1, wherein the residual moisture is equal to or lower than 5% in weight relative to the weight of the inhalable particles.

3. The inhalable particles according to any of the claims 1-2, wherein the indacaterol compound is indacaterol maleate.

4. The inhalable particles according to any of the claims 1-3, wherein the tiotropium compound is tiotropium bromide.

5. The inhalable particles according to any of the claims 1-4, which is a matrix in which all the components are in amorphous state and are dispersed at molecular level.

6. The inhalable particles according to any of the claims 1-4, wherein the sugar derivative is present in an amount up to 85% in weight relative to the weight of the inhalable particles and the tiotropium compound, the indacaterol compound and optionally part of the sugar derivative are dispersed at molecular level at the surface of the sugar derivative.

7. The inhalable particles according to any of the claims 1-5, which is free of sugar derivative, and wherein the weight of the tiotropium compound, expressed as tiotropium base, and the weight of the indacaterol compound, expressed as indacaterol base, relative to the total weight of the inhalable particles are comprised from 0.5 to 50% and from 50 to 99.5% respectively.

8. The inhalable particles according to any of the claims 1-6, wherein the weight of sugar derivative, the weight of the tiotropium compound, expressed as tiotropium base, and the weight of the indacaterol compound, expressed as indacaterol base, relative to the total weight of the inhalable particles are comprised from 20 to 85%, from 0.2 to 10%, and from 10 to 80% respectively.

9. The inhalable particles according to any of the claims 1-8, wherein the sugar derivative is selected from the group consisting of a monosaccharide, a disaccharide, an oligo- or polysaccharide, and a polyalcohol.

10. The inhalable particles according to claim 9, wherein the sugar derivative is lactose.

11. The inhalable particles according to any of claims 1-10, which are spraydried, freeze-dried or vacuum dried particles.

12. A pharmaceutical composition which comprises an effective amount of the inhalable particles as defined in any of the claims 1-11 together with one or more pharmaceutically acceptable excipients or carriers.

13. The pharmaceutical composition according to claim 12, wherein the excipients are coarse excipients having a mean particle size of 15 to 250 $\mu$m.

14. A process for preparing the inhalable particles as defined in any of the claims 1-11, comprising the following steps:

a) If a sugar derivative is present in the final particles, dissolving or dispersing the sugar derivative in a solvent system comprising one or more organic solvents and optionally water to form a solution or a suspension;
b) dissolving a tiotropium compound and an indacaterol compound, in a solvent system comprising one or more organic solvents and optionally water;
c) If a sugar derivative is present in the final particles, mixing the solution or suspension of step a) and the solution of step b); and
d) spray-drying, freeze-drying or vacuum drying the solution or suspension of steps b) or c) to obtain the desired inhalable particles.

15. Inhalable particles as defined in any of the claims 1-11, for use in the treatment of asthma or chronic obstructive pulmonary disease.

**Patentansprüche**

1. Inhalierbare Partikeln bestehend aus einer innigen Mischung, welche aus Folgendem besteht:

   a) einer amorphen Tiotropium-Verbindung,
   b) einer amorphen Indacaterol-Verbindung, und
   c) einem Zuckerderivat,

   wobei das Gewicht des Zuckerderivats von 0 bis 85% bezogen auf das Gewicht der inhalierbaren Partikeln reicht.

2. Die inhalierbaren Partikeln nach Anspruch 1, wobei die Restfeuchtigkeit 5 Gew.-% oder weniger bezogen auf das Gewicht der inhalierbaren Partikeln beträgt.

3. Die inhalierbaren Partikeln nach einem der Ansprüche 1-2, wobei die Indacaterol-Verbindung Indacaterolmaleat ist.

4. Die inhalierbaren Partikeln nach einem der Ansprüche 1-3, wobei die Tiotropium-Verbindung Tiotropiumbromid ist.

5. Die inhalierbaren Partikeln nach einem der Ansprüche 1-4, welche eine Matrix ist, in der alle Bestandteile in einem amorphen Zustand und dispergiert auf molekularer Ebene sind.

6. Die inhalierbaren Partikeln nach einem der Ansprüche 1-4, wobei das Zuckerderivat in einer Menge von bis 85 Gew.-% bezogen auf das Gewicht der inhalierbaren Partikeln ist und die Tiotropium-Verbindung, die Indacaterol-Verbindung und wahlweise ein Teil des Zuckerderivats auf molekularer Ebene auf der Oberfläche des Zuckerderivats dispergiert sind.

7. Die inhalierbaren Partikeln nach einem der Ansprüche 1-5, welche frei von Zuckerderivat sind, und wobei das Gewicht der Tiotropium-Verbindung, ausgedrückt als Tiotropiumbasis, und das Gewicht der Indacaterol-Verbindung, ausgedrückt als Indacaterolbasis, bezogen auf das gesamte Gewicht der inhalierbaren Partikeln jeweils von 0,5 bis 50% und von 50 bis 99,5% reichen.

8. Die inhalierbaren Partikeln nach einem der Ansprüche 1-6, wobei das Gewicht des Zuckerderivats, das Gewicht der Tiotropium-Verbindung, ausgedrückt als Tiotropiumbasis, und das Gewicht der Indacaterol-Verbindung, ausgedrückt als Indacaterolbasis, bezogen auf das gesamte Gewicht der inhalierbaren Partikeln jeweils von 20 bis 85%, von 0,2 bis 10% und von 10 bis 80% reichen.

9. Die inhalierbaren Partikeln nach einem der Ansprüche 1-8, wobei das Zuckerderivat ausgewählt aus der Gruppe bestehend aus einem Monosaccharid, einem Disaccharid, einem Oligo- oder einem Polysaccharid und einem Polyalkohol ist.

10. Die inhalierbaren Partikeln nach Anspruch 9, wobei das Zuckerderivat Laktose ist.

11. Die inhalierbaren Partikeln nach einem der Ansprüche 1-10, welche sprühgetrocknete, gefriergetrocknete oder vakuumgetrocknete Partikeln sind.

12. Eine pharmazeutische Zusammensetzung, welche eine wirksame Menge der inhalierbaren Partikeln wie in einem der Ansprüche 1-11 definiert zusammen mit einem oder mehreren pharmazeutisch akzeptablen Hilfsstoffen oder Trägerstoffen umfasst.

13. Die pharmazeutische Zusammensetzung nach Anspruch 12, wobei die Hilfsstoffe grobe Hilfsstoffe sind, die eine durchschnittliche Partikelgröße von 15 bis 250 μm haben.

14. Ein Verfahren zur Herstellung der inhalierbaren Partikeln wie in einem der Ansprüche 1-11 definiert, umfassend die folgenden Schritte:

   a) wenn ein Zuckerderivat in den Endpartikeln anwesend ist, das Lösen oder das Dispergieren des Zuckerderivats in einem Lösungsmittelsystem umfassend ein oder mehrere organische Lösungsmittel und wahlweise Wasser, um eine Lösung oder eine Suspension zu bilden;
   b) das Lösen von einer Tiotropium-Verbindung und von einer Indacaterol-Verbindung in einem Lösungsmittelsystem umfassend ein oder mehrere organische Lösungsmittel und wahlweise Wasser;
c) wenn ein Zuckerderivat in den Endpartikeln anwesend ist, das Mischen der Lösung bzw. Suspension des Schritts a) und der Lösung des Schritts b); und
d) das Sprühtrocknen, Gefriertrocknen oder Trocknen unter Vakuum der Lösung bzw. Suspension der Schritte b) oder c), um die gewünschten inhalierbaren Partikeln zu erhalten.

**15.** Inhalierbare Partikeln wie in einem der Ansprüche 1-11 definiert, zur Verwendung in der Behandlung von Asthma oder von einer chronisch obstruktiven Lungenerkrankung.

**Revendications**

**1.** Particules inhalables constituées d'un mélange intime qui se compose de :

a) un composé de tiotropium amorphe,
b) un composé d'indacatérol amorphe, et
c) un dérivé de sucre,

dans lesquelles le poids du dérivé de sucre est compris de 0 à 85 % par rapport au poids des particules inhalables.

**2.** Les particules inhalables selon la revendication 1, dans lesquelles l'humidité résiduelle est égale à ou inférieure à 5 % en poids par rapport au poids des particules inhalables.

**3.** Les particules inhalables selon l'une quelconque des revendications 1-2, dans lesquelles le composé d'indacatérol est le maléate d'indacatérol.

**4.** Les particules inhalables selon l'une quelconque des revendications 1-3, dans lesquelles le composé de tiotropium est le bromure de tiotropium.

**5.** Les particules inhalables selon l'une quelconque des revendications 1-4, qui sont une matrice dans laquelle tous les composants sont à l'état amorphe et dispersés à niveau moléculaire.

**6.** Les particules inhalables selon l'une quelconque des revendications 1-4, dans lesquelles le dérivé de sucre est présent dans une quantité de jusqu'à 85 % en poids par rapport au poids des particules inhalables et le composé de tiotropium, le composé d'indacatérol et facultativement une partie du dérivé de sucre sont dispersés à niveau moléculaire dans la surface du dérivé de sucre.

**7.** Les particules inhalables selon l'une quelconque des revendications 1-5, qui sont exemptes de dérivé de sucre, et dans lesquelles le poids du composé de tiotropium, exprimé comme base de tiotropium, et le poids du composé d'indacatérol, exprimé comme base d'indacatérol, par rapport au poids total des particules inhalables sont compris de 0,5 à 50 % et de 50 à 99,5 %, respectivement.

**8.** Les particules inhalables selon l'une quelconque des revendications 1-6, dans lesquelles le poids de dérivé de sucre, le poids du composé de tiotropium, exprimé comme base de tiotropium, et le poids du composé d'indacatérol, exprimé comme base d'indacatérol, par rapport au poids total des particules inhalables sont compris de 20 à 85 %, de 0,2 à 10 % et de 10 à 80 %, respectivement.

**9.** Les particules inhalables selon l'une quelconque des revendications 1-8, dans lesquelles le dérivé de sucre est choisi dans le groupe constitué d'un monosaccharide, un disaccharide, un oligo- ou polysaccharide, et un polyalcool.

**10.** Les particules inhalables selon la revendication 9, dans lesquelles le dérivé de sucre est la lactose.

**11.** Les particules inhalables selon l'une quelconque des revendications 1-10, qui sont des particules séchées par pulvérisation, lyophilisées ou séchées sous vide.

**12.** Une composition pharmaceutique qui comprend une quantité efficace des particules inhalables telles que définies dans l'une quelconque des revendications 1-11 avec un ou plusieurs excipients ou véhicules pharmaceutiquement acceptables.

**13.** La composition pharmaceutique selon la revendication 12, dans laquelle les excipients sont des excipients grossiers ayant une taille des particules moyenne de 15 à 250 μm.

**14.** Un procédé de préparation des particules inhalables telles que définies dans l'une quelconque des revendications 1-11, comprenant les étapes suivantes :

a) si un dérivé de sucre est présent dans les particules finales, dissoudre ou disperser le dérivé de sucre dans un système solvant comprenant un ou plusieurs solvants organiques et facultativement de l'eau pour former une solution ou une suspension;
b) dissoudre un composé de tiotropium et un composé d'indacatérol, dans un système solvant comprenant un ou plusieurs solvants organiques et facultativement de l'eau;
c) si un dérivé de sucre est présent dans les particules finales, mélanger la solution ou la suspension de l'étape a) et la solution de l'étape b); et
d) sécher par pulvérisation, lyophiliser ou sécher sous vide la solution ou suspension des étapes b) ou c) afin d'obtenir les particules inhalables désirées.

**15.** Particules inhalables telles que définies dans l'une quelconque des revendications 1-11, pour l'utilisation dans le traitement de l'asthme ou d'une bronchopneumopathie chronique obstructive.

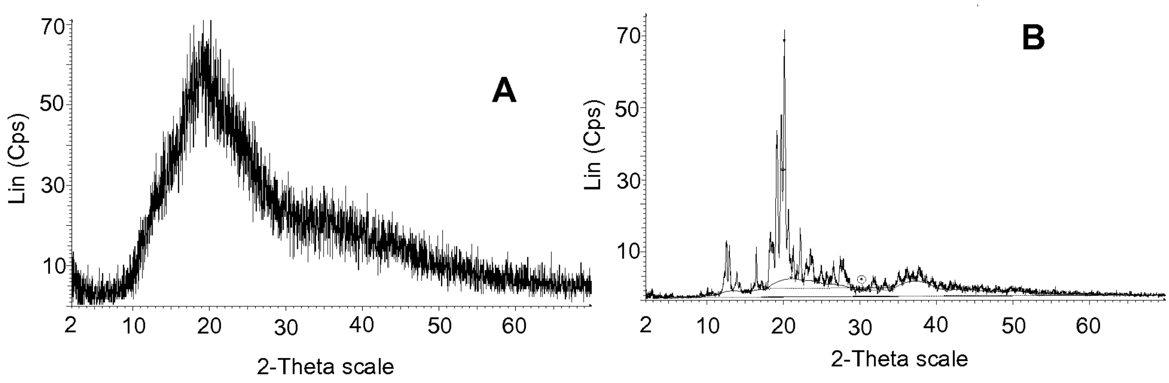


FIG. 1

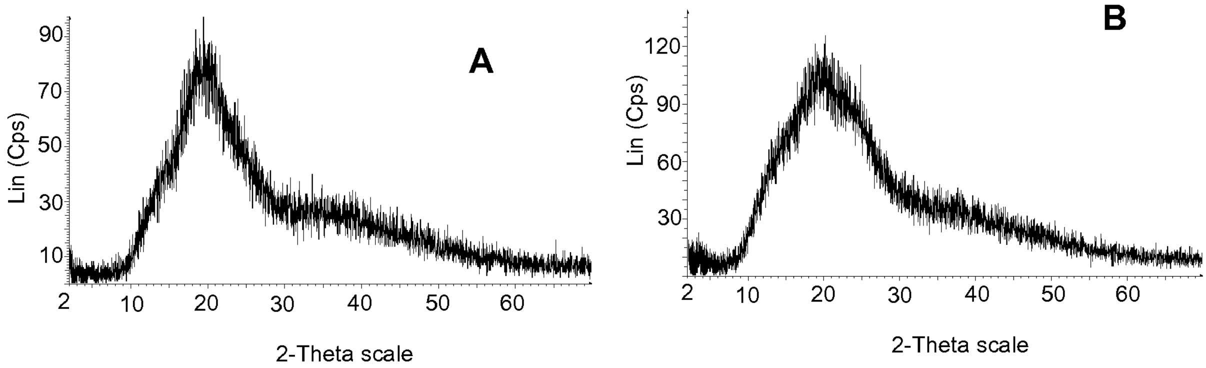


FIG. 2

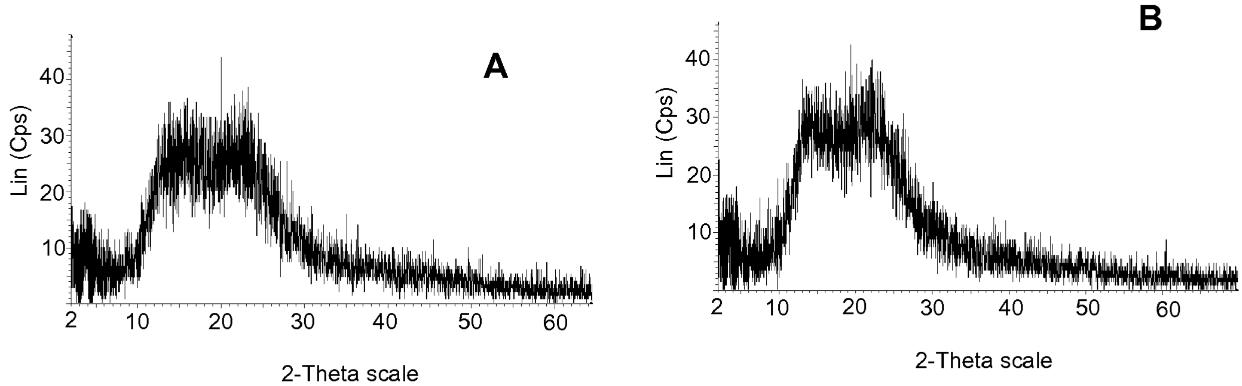


FIG. 3

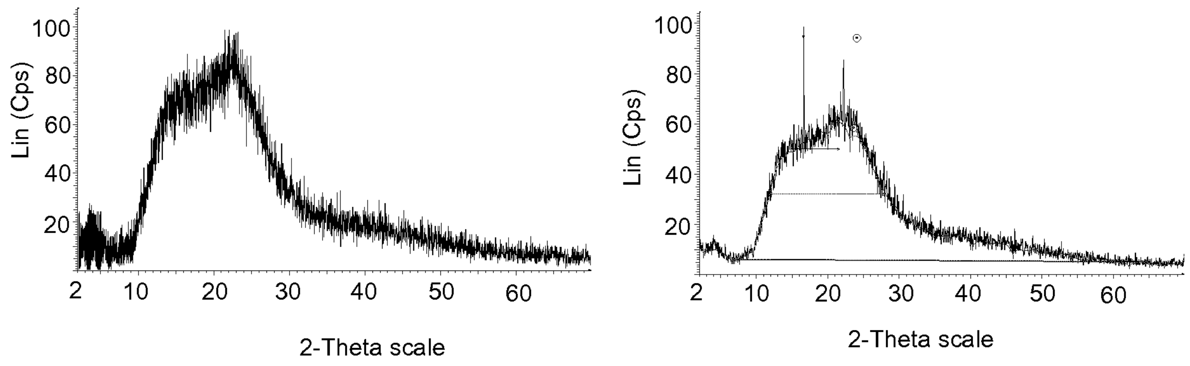


FIG. 4

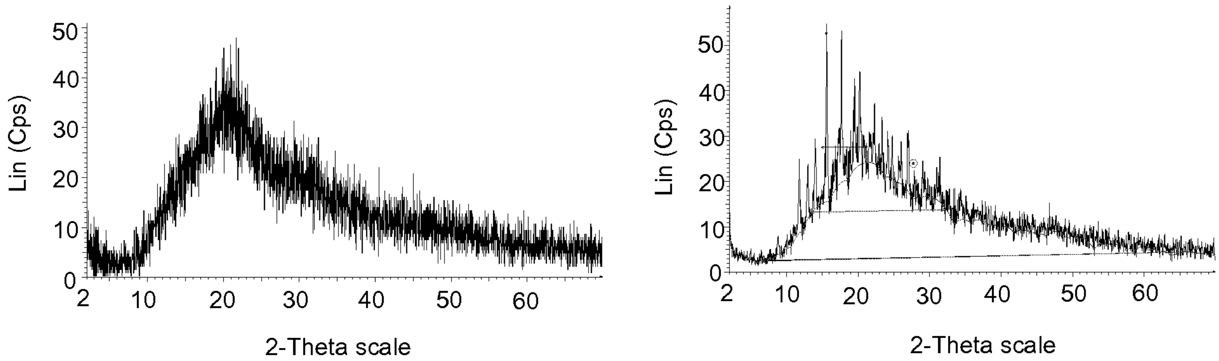


FIG. 5

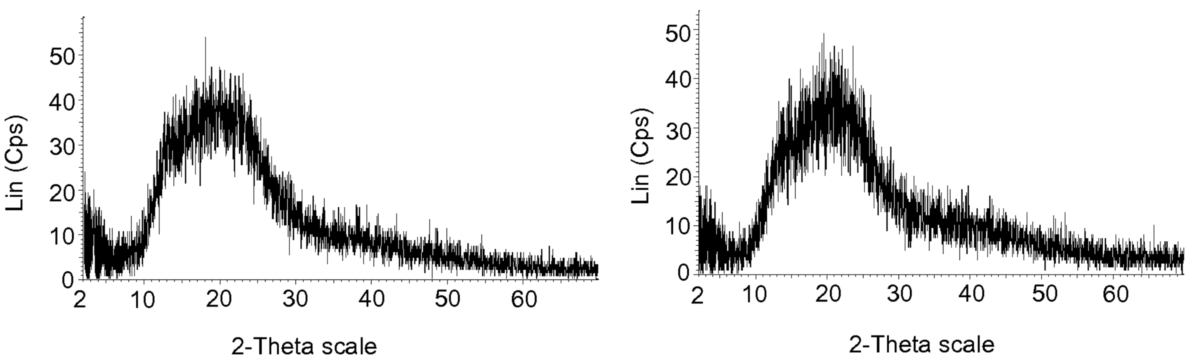


FIG. 6

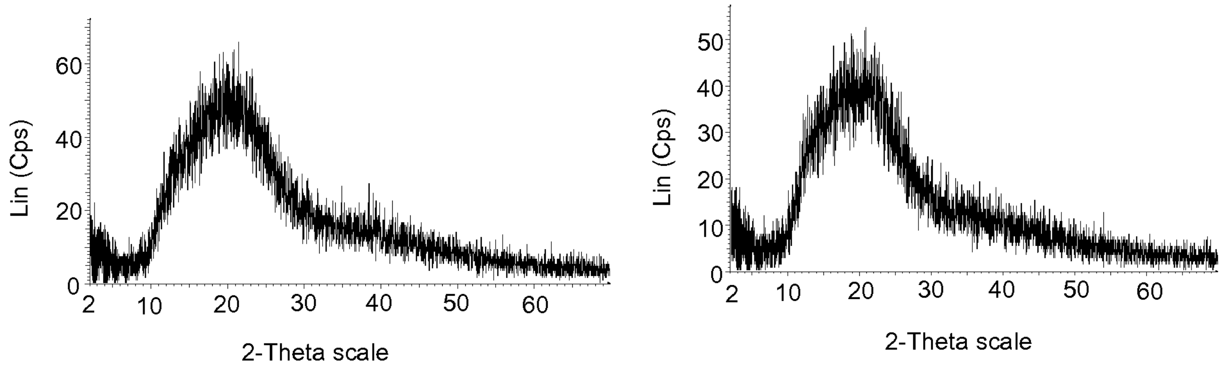


FIG. 7

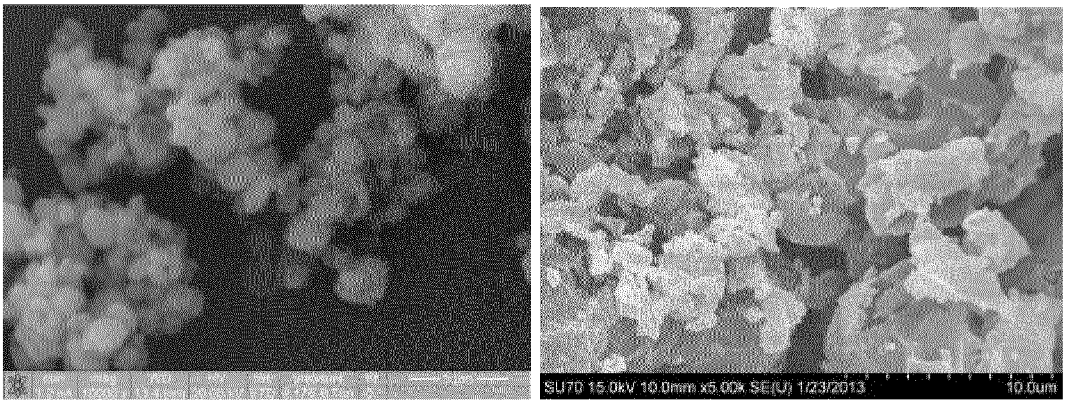


FIG. 8

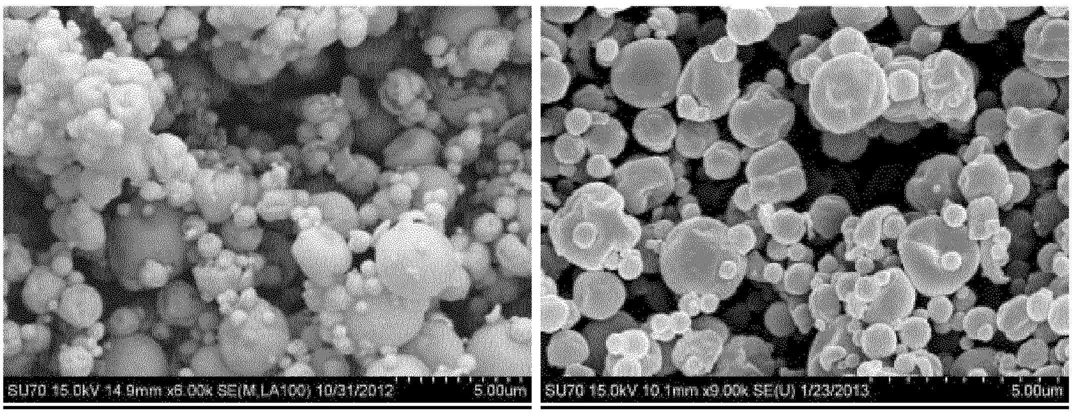


FIG. 9

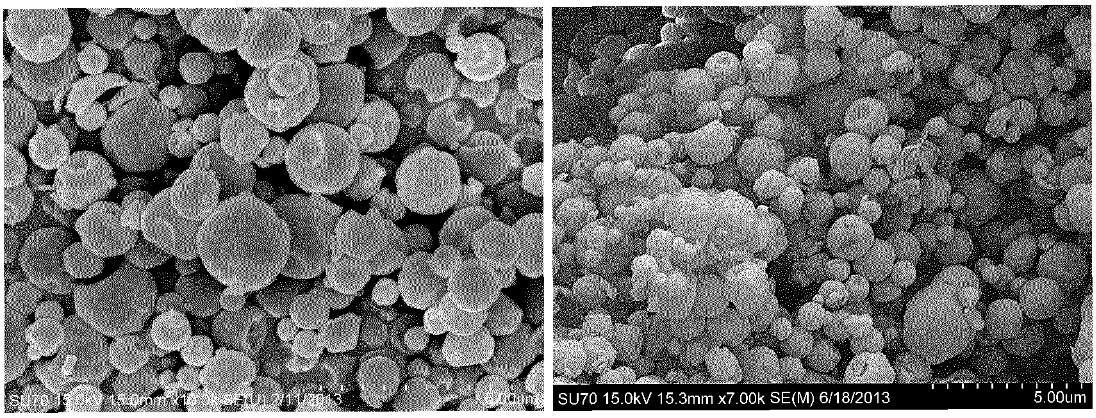


FIG. 10

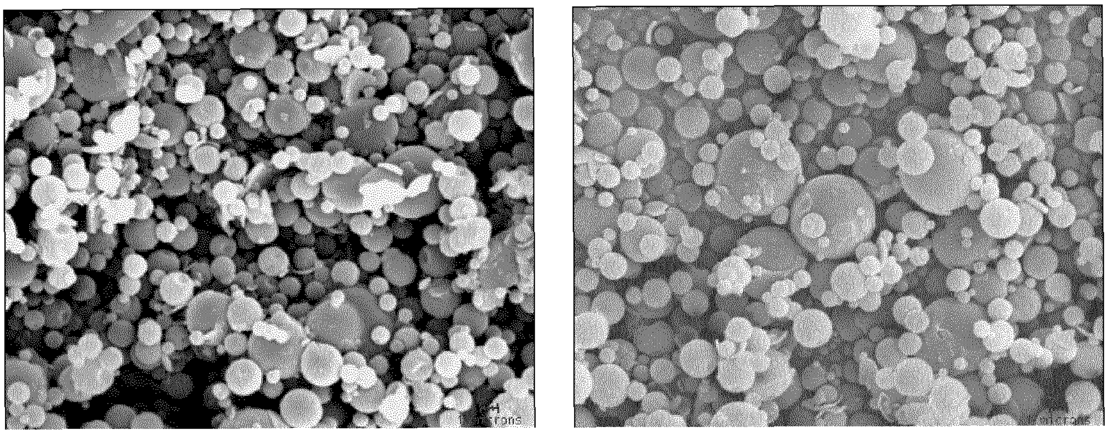

FIG. 11

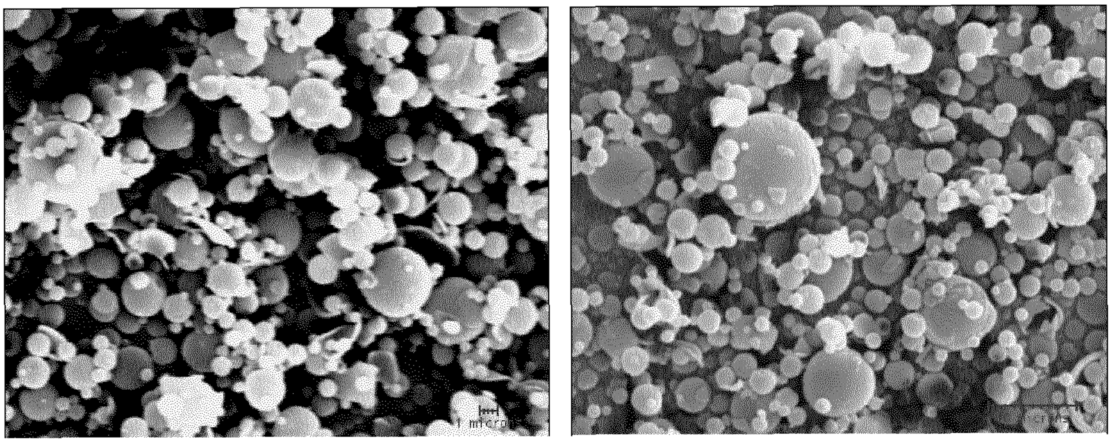


FIG. 12

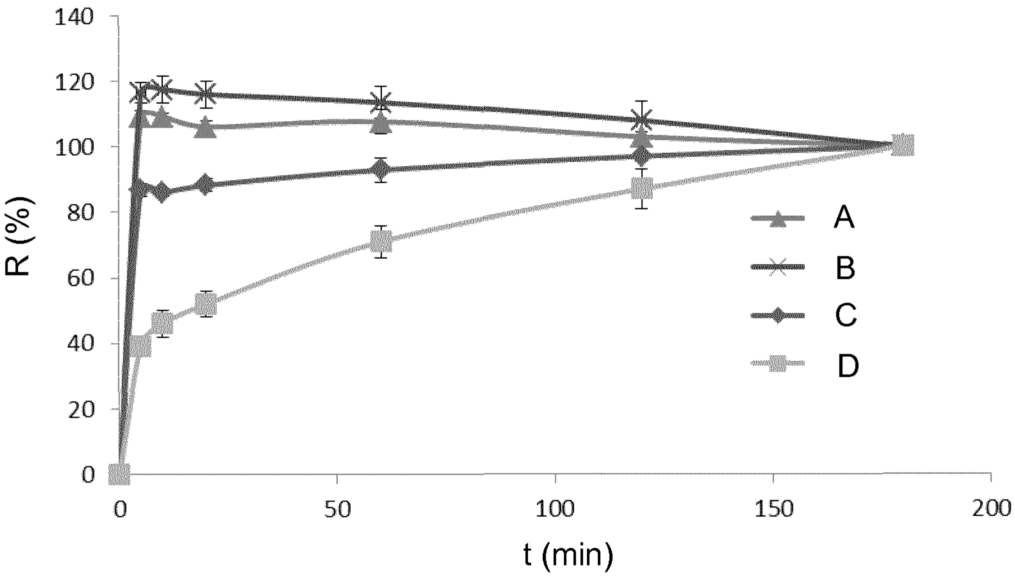
140
120
100
80
60
40
20
0
R (%)
0
50
100
150
200
t (min)
A
B
C
D

FIG. 13

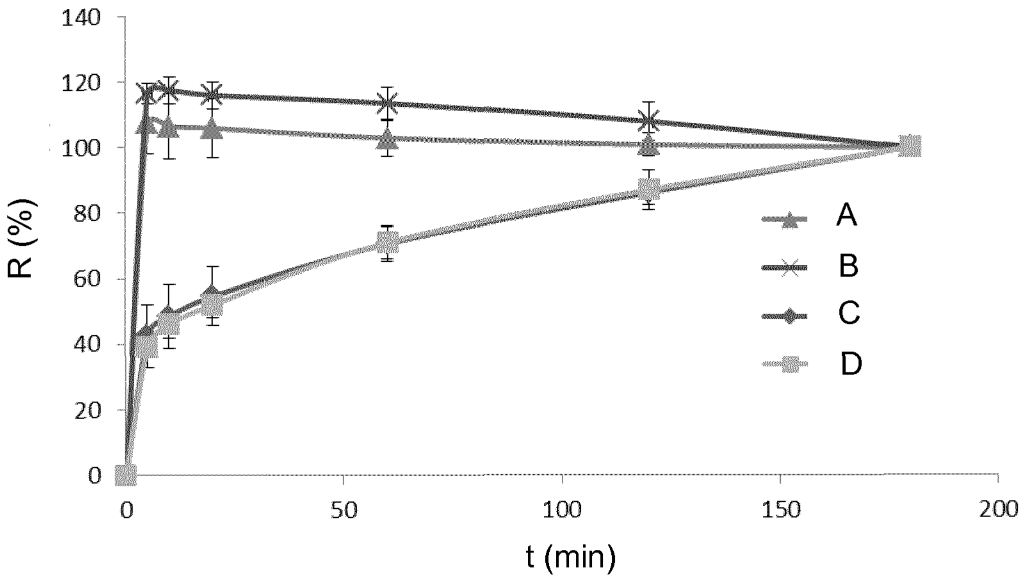
140
120
100
80
60
40
20
0
R (%)
0
50
100
150
200
t (min)
A
B
C
D

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5610163 A
- US 6878721 B
- US 20040132759 A
- WO 2012007729 A
- WO 2013021199 A
- WO 2012106575 A